**Europäisches Patentamt**

(19) **European Patent Office**

**Office européen des brevets**

(11) Numéro de publication: **0 110 791**
**B1**

# FASCICULE DE BREVET EUROPEEN

(45) Date de publication du fascicule du brevet: **25.04.90**

(21) Numéro de dépôt: **83402310.3**

(22) Date de dépôt: **30.11.83**

(60) **Demande divisionnaire 89102888.8 déposée le 30/11/83.**

(51) Int. Cl.⁵: **C 12 N 15/00,** C 12 P 21/02, C 07 K 13/00, C 07 H 21/04, A 61 K 39/13, C 12 Q 1/68

(54) **Peptides comportant un site immunogène du poliovirus et ADNs contenant des séquences nucléotidiques codant pour ces peptides.**

(30) Priorité: **30.11.82 FR 8220115**
**29.06.83 FR 8310778**

(43) Date de publication de la demande:
**13.06.84 Bulletin 84/24**

(45) Mention de la délivrance du brevet:
**25.04.90 Bulletin 90/17**

(84) Etats contractants désignés:
**AT BE CH DE FR GB IT LI LU NL SE**

(56) Documents cités:
**EP-A-0 086 707**
**WO-A-82/03632**
**WO-A-82/04067**

(73) Titulaire: **INSTITUT PASTEUR**
**25-28, rue du Docteur Roux**
**F-75724 Paris Cédex 15 (FR)**

(72) Inventeur: **Girard, Marc**
**6, rue César Franck**
**F-75015 Paris (FR)**
Inventeur: **Van der Werf, Sylvie**
**3, Villa Grenelle**
**F-75015 Paris (FR)**

(74) Mandataire: **Gutmann, Ernest et al**
**S.C. Ernest Gutmann - Yves Plasseraud 67, boulevard Haussmann**
**F-75008 Paris (FR)**

Courier Press, Leamington Spa, England.

EP 0 110 791 B1

# EP 0 110 791 B1

**Description**

L'invention est relative à des peptides comportant un site immunogène du poliovirus et des fragments d'ADN contenant des séquences nucléotidiques codant pour ces peptides. L'invention concerne encore les principes vaccinants mettant en jeu de tels peptides, ces principes étant aptes à induire chez l'hôte, homme ou animal, la production d'anticorps actifs non seulement contre euxmêmes, mais encore contre les poliovirus infectieux entiers.

On a déjà décrit dans la demande de brevet français no 82 02013 déposée le 8 février 1982 (FR—A—2521165) des fragments d'ADN codant pour un peptide immunogène susceptible d'induire *in vivo* la synthèse d'anticorps antipoliovirus. Ces fragments d'ADN possèdent une longueur n'excédant pas celle d'un fragment d'ADN comportant de l'ordre de 1,2 kb (kilopaire de bases). Ces fragments sont plus particulièrement caractérisés en ce qu'ils contiennent une séquence nucléotidique codant pour la protéine VP-1, laquelle s'est avérée porter des déterminants antigéniques essentiels intervenant au niveau de l'immunogénicité du poliovirus infectieux correspondant. En effet ce peptide est susceptible de former des complexes antigène-anticorps avec des sérums neutralisants monoclonaux ou polyclonaux obtenus à partir d'animaux auxquels avait été injecté du poliovirus entier (sérum de spécificité D).

Des séquences types d'ADN codant pour des peptides immunogènes du type sus-indiqué sont illustrées dans la succession des figures 1 et 2 ci-annexées, pour l'un d'entre eux, et dans la succession des figures 3 et 4, également ci-annexées, pour un autre fragment d'ADN contenant la susdite séquence. Les emplacements de certains sites de restriction auxquels il sera fait référence dans ce qui suit sont également indiqués dans ces figures. Les numérotations des nucléotides successifs intervenant dans la constitution de ces ADN s'effectuent à partir de l'extrémité 5'. Pour ce qui est de la constitution de l'ADN clonable du poliovirus dont sont issus les susdits ADN on se référera à l'article de Sylvie VAN DER WERF et autres auteurs, intitulé "Molecular cloning of the genome of poliovirus" dans Proc. Nat. Acad. Sci. USA, vol. 78, no 10, pp. 59—83, 59—87, oct. 1981.

L'invention découle de la découverte que des peptides correspondant à des séquences d'ADN contenues dans les précédentes, mais beaucoup plus petites que celles-ci, portaient néanmoins des déterminants antigéniques permettant leur utilisation dans la constitution de principes vaccinants efficaces contre les poliovirus correspondants. Parmi les peptides en question, on peut en isoler certains dont la taille est suffisamment réduite pour qu'ils soient directement accessibles par synthèse chimique.

L'invention fournit en outre la technique permettant la détermination, au sein des ADN de tailles relativement importantes qui font l'objet de la demande de brevet français no 82 02013, de celles des séquences d'ADN beaucoup plus petites auxquelles correspondent des peptides présentant des déterminants ou sites antigéniques les rendant aptes à être mis en oeuvre dans la production de principes vaccinants à l'égard de poliovirus correspondants entiers et infectieux.

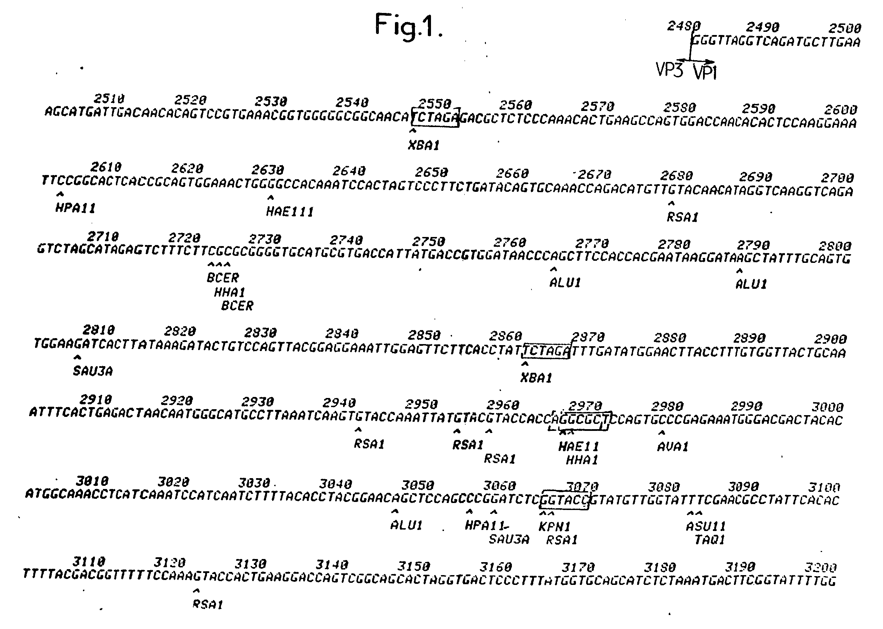

A cet égard, la plus longue des séquences d'ADN selon l'invention est constituée par le fragment délimité à ses extrémitées opposées par les sites Xbal situés dans les régions définies par les positions 2546 et 2861 de la fig. 1.

L'invention concerne plus particulièrement encore celles des séquences d'ADN contenues au sein de la précédente et qui codent un peptide susceptible d'être reconnu par des anticorps monoclonaux actifs à la fois contre les particules "C" et "D" originaires d'un même poliovirus et contre le polypeptide structural VP-1 de la capside du même poliovirus. C'est de ce type d'anticorps monoclonaux qu'il s'agira en toutes circonstances dans l'exposé qui suit, sauf lorsqu'il sera spécifié autrement.

De tels anticorps sont obtenus à partir d'hydridomes, lesquels ont été obtenus par la réalisation d'un fusion entre des cellules spléniques d'un animal préalablement immunisé par un virus ou virion présentant une antigénicité "C" (obtenu notamment par chauffage pendant 1 heure à 56°C du poliovirus infectieux correspondant à antigénicité "D") et des cellules myélomateuses appropriées, en mettant en oeuvre une technique en soi connue, par la culture des hydrides ou clones cellulaires obtenus et par la sélection des clones qui s'avèrent produire des anticorps monoclonaux actifs à la fois contre les virons à antigénicité "C", les virions homologues à antigénicité "D" et contre la protéine correspondante VP-1. Les poliovirus dont il est question sont avantageusement du type 1 (Mahoney). De tels anticorps monoclonaux (qui seront encore désignés dans ce qui suit sous l'expression "anticorps CD-VP-1" (ou "C3")), les hybrides cellulaires susceptibles de les produire et un procédé d'obtention de ces derniers ont été décrits dans la demande de brevet français no 82 19338 (FR—A—2536414) déposée le 18 novembre 1982. Deux des hybrides cellulaires formés ont été déposés dans la Collection Nationale de Culture de Micro-Organismes de l'INSTITUT PASTEUR de Paris (C.N.C.M.), respectivement sous les no I-208 et no I-209.

Cette séquence d'ADN selon l'invention présente la structure suivante:

2

```
            TCT AGA GAC GCT CTC CCA AAC ACT GAA
        GCC AGT GGA CCA ACA CAC TCC AAG GAA ATT
        CCG GCA CTC ACC GCA GTG GAA ACT GGG GCC
        ACA AAT CCA CTA GTC CCT TCT GAT ACA GTG
        CAA ACC AGA CAT GTT GTA CAA CAT AGG TCA
        AGG TCA GAG TCT AGC ATA GAG TCT TTC TTC
        GCG CGG GGT GCA TGC GTG ACC ATT ATG ACC
        GTG GAT AAC CCA GCT TCC ACC ACG AAT AAG
        GAT AAG CTA TTT GCA GTG TGG AAG ATC ACT
        TAT AAA GAT ACT GTC CAG TTA CGG AGG AAA
        TTG GAG TTC TTC ACC TAT TCT.
```

L'invention concerne naturellement toute séquence d'ADN codant pour un peptide ayant les propriétés immunogènes semblables à celles du peptide codé par la susdite séquence nucléotidique. En particulier tout triplet de cette séquence peut être remplacé, soit par un triplet distinct codant pour le même acide aminé ou pour un acide aminé distinct, dans la mesure où la substitution du second au premier dans la peptide codé par la séquence d'ADN en question, n'altérera pas fondamentalement les propriétés immunogènes du peptide codé par la séquence d'ADN ainsi modifiée. En particulier, l'invention concerne toute séquence d'ADN de ce type codant pour un peptide susceptible d'être reconnu par l'anticorps C3 sus-indiqué.

L'invention concerne encore toute séquence nulcéotidique de plus faible longueur contenue dans la précédente, dès lors qu'elle code pour un peptide toujours encore susceptible d'être reconnu par l'anticorps C3.

Parmi les séquences d'ADN entrant dans la champ de l'invention, figurent celles qui contiennent les séquences nuclétidiques codant pour la séquence peptidique His 65—Phe 105 définie ci-après et plus particulièrement encore la séquence nucléotidique 2671—2792 du gène codant pour le polypeptide de structure VP-1 du poliovirus de la fig. 1.

D'autres séquences préférés d'ADN entrant dans le champ de l'invention sont celles qui codent pour les séquences peptidiques His 65—Ile 110 définies ci-après, et plus particulièrement encore la séquence nucléotidique Pro 95—Ile 110 du même gène.

L'invention concerne naturellement les polypeptides contenant les séquences peptidiques codées par les susdites séquences d'ADN. Elle concerne en particulier la séquence de formule:

```
            Ser Arg Asp Ala Leu Pro Asn Thr Glu
        Ala Ser Gly Pro Thr His Ser Lys Glu Ile
        Pro Ala Leu Thr Ala Val Glu Thr Gly Ala
        Thr Asn Pro Leu Val Pro Ser Asp Thr Val
        Gln Thr Arg His Val Val Gln His Arg Ser
        Arg Ser Glu Ser Ser Ile Glu Ser Phe Phe
        Ala Arg Gly Ala Cys Val Thr Ile Met Thr
        Val Asp Asn Pro Ala Ser Thr Thr Asn Lys
        Asp Lys Leu Phe Ala Val Trp Lys Ile Thr
        Tyr Lys Asp Thr Val Gln Leu Arg Arg Lys
        Leu Glu Phe Phe Thr Tyr Ser.
```

L'invention concerne également tout peptide présentant des propriétés immunogènes équivalentes dans les conditions qui ont déjà été indiquées en rapport avec les peptides codés par les séquences d'ADN ci-dessus définies. A ce titre l'invention concerne plus particulièrement la sequence suivante, ci-après dite "séquences His 65—Phe 105".

# EP 0 110 791 B1

```
                                 His Val Val Gln His
       Arg Ser Arg Ser Glu Ser Ser Ile Glu Ser
       70
       Phe Phe Ala Arg Gly Ala Cys Val Thr Ile
       80
       Met Thr Val Asp Asn Pro Ala Ser Thr Thr
       90
       Asn Lys Asp Lys Leu Phe
       100
```

ou dite ci-après "séquence His 65—Ile 110".

```
                                 His Val Val Gln His
       Arg Ser Arg Ser Glu Ser Ser Ile Glu Ser
       70
       Phe Phe Ala Arg Gly Ala Cys Val Thr Ile
       80
       Met Thr Val Asp Asn Pro Ala Ser Thr Thr
       90
       Asn Lys Asp Lys Leu Phe Ala Val Trp Lys
       100
       Ile
       110
```

L'invention concerne plus particulièrement encore ceux des peptides qui contiennent la séquence peptidique suivante, ci-après dénommée Asp 93—Leu 104: Asp Asn Pro Ala Ser Thr Thr Asn Lys Asp Lys Leu.

L'invention concerne concerne naturellement aussi les vecteurs, notamment du type plasmides ou phages, contenant un insérat formé par l'une quelconque des séquences d'ADN, telles qu'elles ont été définies ci-dessus. Ces vecteurs modifiés peuvent être mis en oeuvre dans la transformation d'organismes cellulaires ou de micro-organismes appropriès, en vue d'induire la production par celle-ci de polypeptide, le cas échéant hybrides, contenant une séquence peptidique susceptible d'être reconnue par les anticorps monoclonaux CD-PV1 ou C3 ou d'autres anticorps reconnaissant le virus infectieux. Ces polypeptides, le cas échéant hybrides, font également partie de l'invention.

L'invention fournit encore un procédé permettant l'identification, au sein d'une séquence d'ADN normalement contenue à l'intérieur de l'ADN d'un poliovirus déterminé, de celles des séquences plus petites qui sont susceptibles de coder pour un peptide immunogène ou susceptibles d'être mises en oeuvre dans la fabrication d'un principe immunogène permettant la production d'anticorps actifs contre le poliovirus entier correspondant.

Ce procédé est essentiellement caractérisé en ce que, partant d'un plasmide contenant un insérat formé par une séquence initiale reconnue comme devant contenir une séquence plus petite susceptible de coder pour un peptide immunogène ou susceptible d'entrer dans la constitution d'un principe immunogène, on linéarise ledit plasmide au niveau d'un site de restriction extérieur à ladite séquence plus petite, on émonde de façon contrôlée le plasmide linéarisé avec une enzyme exonucléolytique, telle que l'enzyme Bal 31, on recircularise le plasmide au moyen d'une ADN-ligase, on transforme un micro-organisme approprié, lui-même transformable par le vecteur correspondant et capable d'exprimer l'insérat contenue dans celui-ci, et on détecte parmi les produits d'expression de ce micro-organisme l'éventuelle présence d'un peptide susceptible de porter le site immunogène du genre en question, par mise en contact desdits produits d'expression avec un anticorps monoclonal CD-PV1, le cycle d'opérations qui vient d'être défini étant répété jusqu'à la disparition de la détection dudit peptide immunogène parmi les produits d'expression dudit micro-organisme transformé par le dernier plasmide recircularisé.

Il est possible, au terme de chacun des cycles du procédé sus-défini, par exemple par comparaison des cartes de restriction du plasmide avant et après la susdite opération d'émondage, de déterminer celles des séquences d'ADN qui ont été éliminées entre deux émondages successifs et, par conséquent, lorsque cesse la possibilité de détection d'un peptide immunogène dans les conditions sus-indiquées, de corréler ce résultat à l'une des séquences supprimée au cours de l'opération d'émondage précédente, cette séquence supprimée d'ADN codant pour ledit peptide immunogène. La structure de la séquence supprimée (ou des séquences supprimées) peut naturellement découler des déterminations de séquences nucléotidiques d'extrémités effectuées avant et après l'émondage considéré.

Un tel principe sera illustré dans l'un des exemples de mise en oeuvre de l'invention dont la description suit. Il sera également fait référence dans ce qui suit aux dessins dans lesquels:

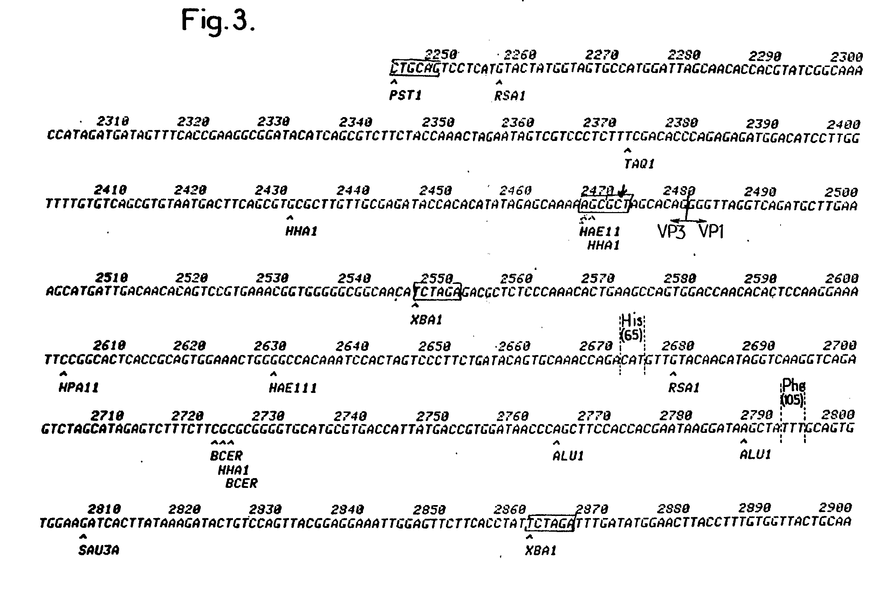

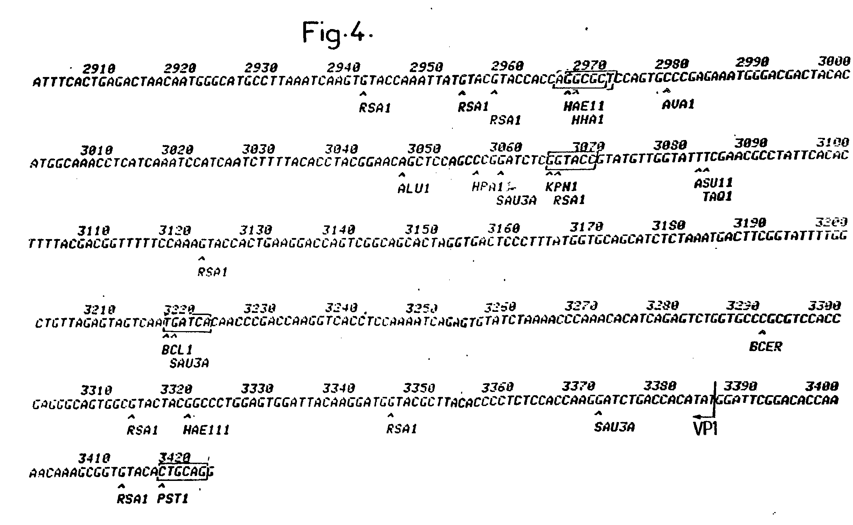

les fig. 1 à 4 correspondent à des séquences déjà définies dans ce qui précède;

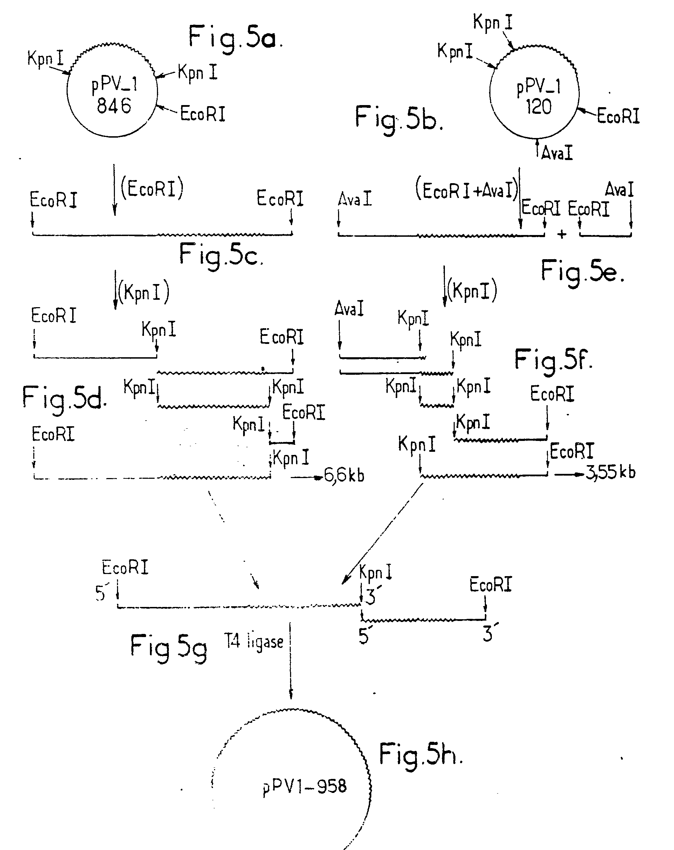

les fig. 5a à 5h schématisent un mode de production d'un précurseur obtenu à partir des clones pPV1-846 et pPV1-120 décrits dans l'article de Sylvie VAN DER WERF et autres auteurs déjà mentionné plus haut;

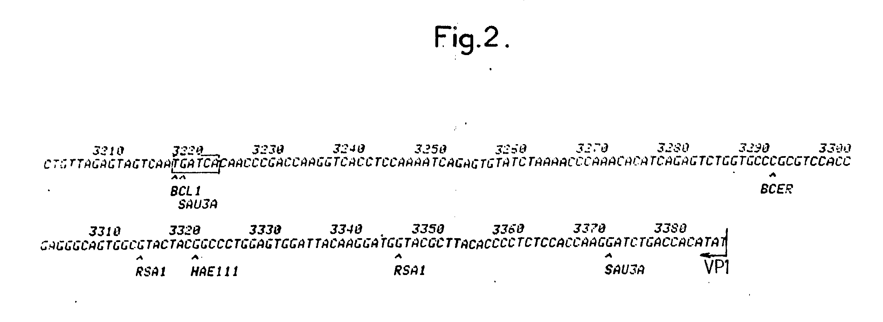

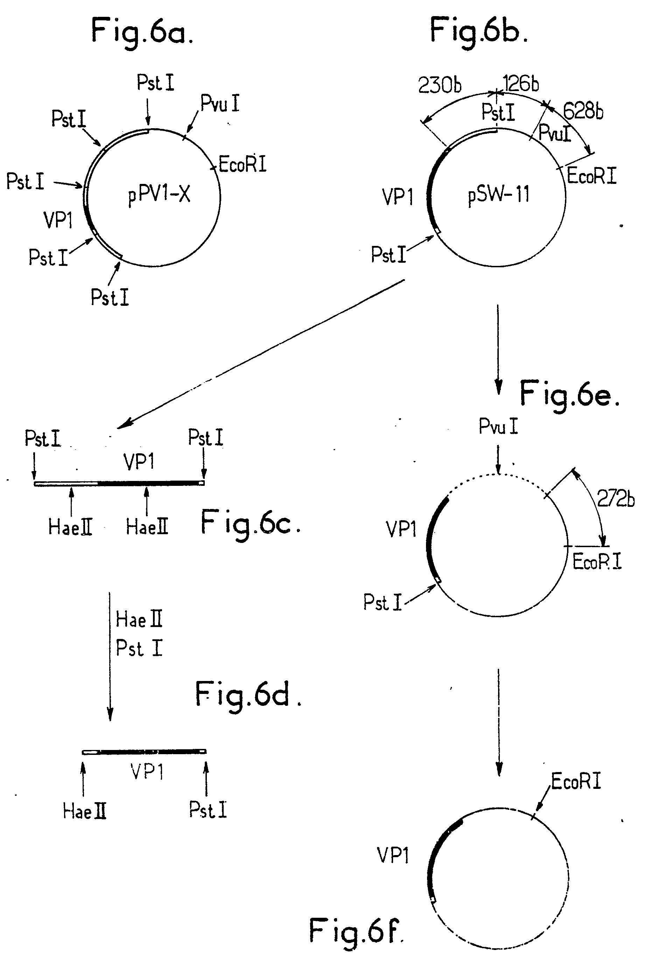

les fig. 6a à 6f représentent schématiquement les étapes d'un mode de production d'un plasmide contenant l'essentiel de l'information génétique de la séquence d'ADN, telle qu'elle résulte des fig. 1 et 2;

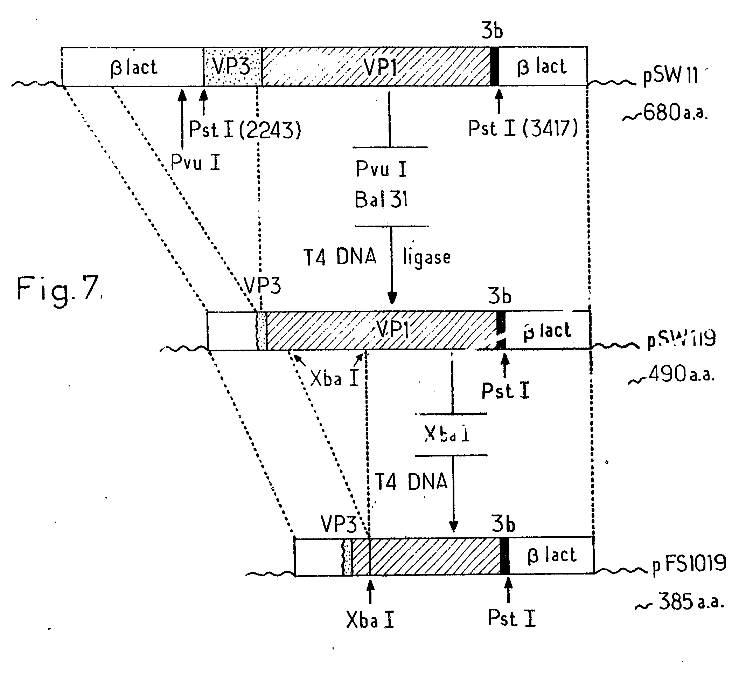

la fig. 7 est une représentation schématique de la production du plasmide précédent et d'une étape supplémentaire mise en jeu dans une première étape de la présente invention, comme il résultera de la description qui suit.

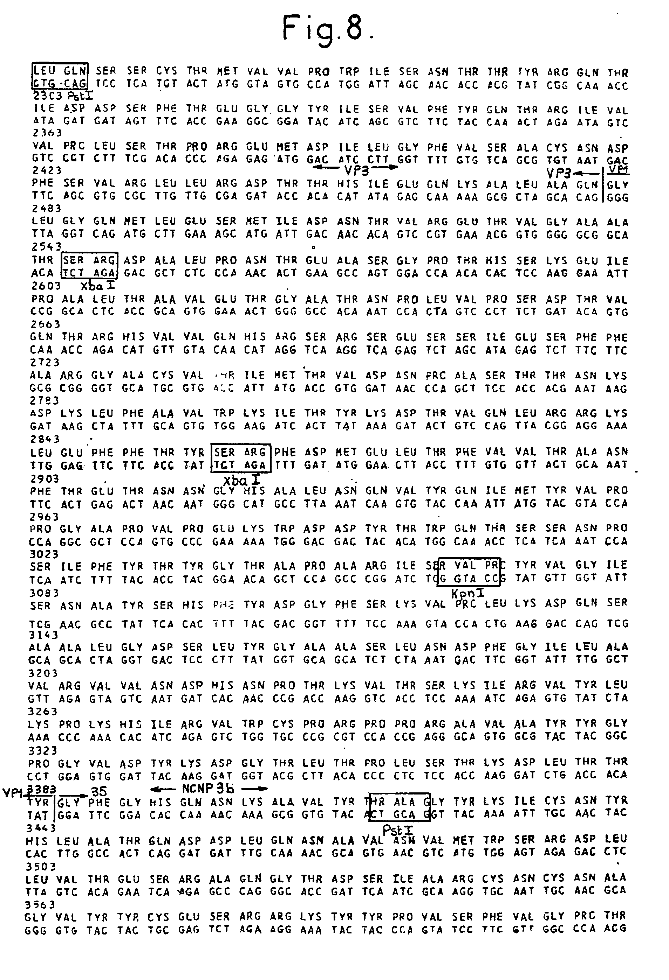

La figure 8 est une nouvelle reproduction de la séquence codant pour VP1, précédée d'une partie de la séquence codant pour VP3 et suivie par une partie de la séquence codant pour NCVP3b. Cette séquence ne se différencie essentiellement des parties de séquences correspondantes apparaissant aux figures 1 à 4 que par la numérotation des nucléotides. Cette numérotation est conforme à celle résultant du "consensus" auquel se réfèrent A. J. DORNER et al dans l'article institulé: "Identification of the initiation site of poliovirus polyprotein synthesis" (Identification du site d'initiation de la synthèse de la polyprotéine de poliovirus) (Journal of Virology, Juin 1982, vol. 42, No 3, pp.1 017 — 1 028).

Cette publication renvoie au projet MOLGEN du système SUMEX AIM de l'Université de Stanford en ce qui concerne les relations à établir entre la numérotation des séquences entièrement publiées et la numérotation adoptée dans la figure 8.

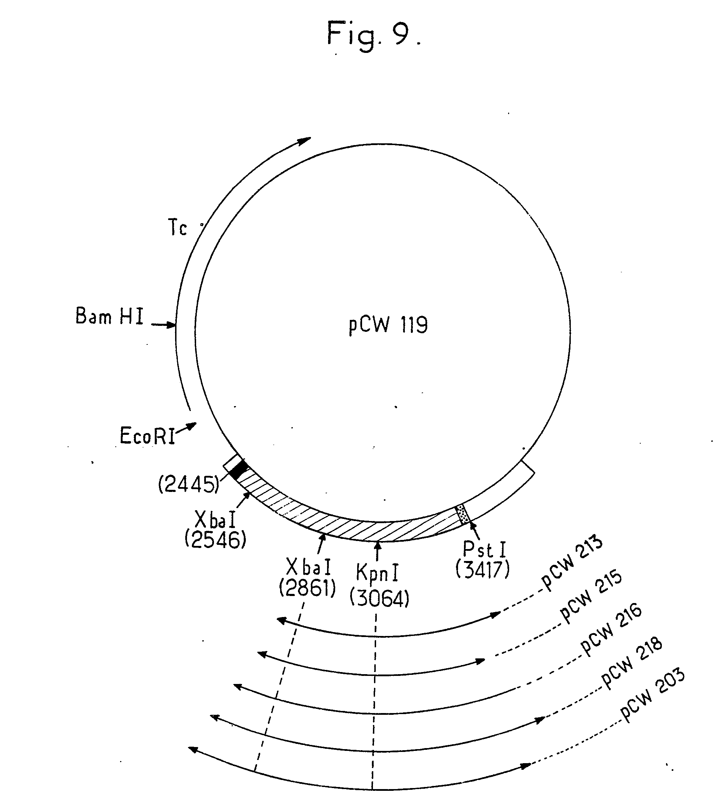

La figure 9 est une représentation schématique du plasmide pCW 119. Elle illustre les positions relatives des délétions introduites dans d'autres plasmides dont question plus loin et dérivés de pCW 119.

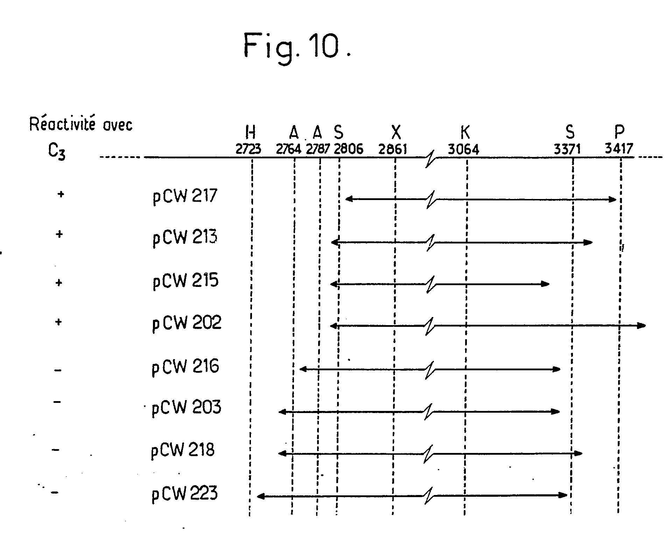

La figure 10 illustre plus spécifiquement encore les positions de ces délétions vis-à-vis des sites de restriction déterminés dans le plasmide pCW 119.

Les techniques des constructions utilisées pour les différents plasmides sont classiques. Les ADN de plasmides ont chaque fois été clivés par les enzymes de restriction dans les conditions prévues par leurs fabricants respectifs. Les fragments d'ADN ont été analysés par électrophorèse dans un gel d'Agarose ou de polyacrylamide. Les extrémités saillantes 3' ont été transformées en extrémités franches par incubation des fragments d'ADN (0,1 mg/ml) avec 100 µ/ml de polymérase ADN I (fragment Klenow) de *E. coli* pendant 1 heure à 37°C dans un milieu 10 mM Tris-HCl, pH 7,5 contenant 10 mM $MgCl_2$, 50 mM NaCl, 1 mM DTT en présence de 0.2 mM des premières paires de nucléotides. La digestion avec la nucléase *Ba* 31 a été réalisé dans un milieu 20 mM $CaCl_2$, 12 mM $MgCl_2$, en mettant en oeuvre un rapport enzyme/DNA de 0,12 u par µg. Après incubation pendant 15 minutes à 30°C, on ajoute de l'EDTA jusqu'à atteindre une concentration de 50 mM et le DNA est extrait avec du phénol et précipité avec de l'éthanol. Les réactions de ligation ont été réalisées dans 20 µl d'un milieu 60 mM Tris-HCl, pH 7,5, 10 mM $MgCl_2$, 10 mM DTT, 1 mM ATP pendant 18 heures à 15°C, en utilisant 1 µ de T4 DNA Ligase par µg de DNA. Les plasmides linéarisés ont, le cas échéant, été traités pendant 30 min. à 68°C avec une phosphatase alkaline bactérienne (0,02 µ par µg DNA) avant ligation avec les fragments appropriés.

## 1. Hydrolyse des DNA clonés par des enzymes de restriction

1.1. L'ADN du plasmide pPVI-846 est hydrolysé complètement par EcoRI. La forme linéaire du DNA plasmidique ainsi obtenue (figure 5c) est hydrolysée par digestion partielle avec Kpn I; les fragments obtenus (figure 5d) sont séparés par électrophorèse en gel d'agarose à 0,7%.

Le fragment de taille 6,6 kbp est sélectionne. Il représente en effet la séquence du plasmide pBR322 du site EcoRI au site Pst I, prolongée de celle du DNA correspondant à la séquence du poliovirus qui s'étend du nucléotide 1 au nucléotide 3064 (2e site Kpn I).

1.2. L'ADN du clone pPVI-120 est hydrolysé dans une digestion complète par Aval et EcoRI formant ainsi 2 fragments de tailles différentes (figure 5e). L'ADN est hydrolysé ensuite partiellement par Kpn I. Les fragments ainsi obtenus (figure 5f) sont séparés par électrophorèse en gel d'agarose à 0,7%.

Le fragment de taille 3,55 kbp est sélectionné. Il représente en effet la séquence du cDNA du poliovirus allant du nucléotide 3064 (2e site Kpn I) au nucléotide 5650 environ, prolongée de celle des 752 paires de bases du segment Pst I-EcoRI du plasmide pBR322.

## 2. Extraction des fragments de DNA des gels

2.1. Les fragments sont visualisés dans les gels par coloration au bromure d'éthidium; ceux de la taille voulue sont extraits des gels par électroélution en sac à dialyse.

2.2. Le matériel ainsi obtenu est purifié et concentré.

3. Recollage des fragments (recombinaison)

Les deux fragments sélectionnés provenant des clones pPVI-846 et pPVI-120 et décrits ci-dessus sont mélangés et recollés à l'aide de la DNA ligase du phage T4. Les bouts collants formés aux points de coupure par EcoRI et KpnI et portés à chaque extrémité des deux fragments favorisent leur recollage et assurent que celui-ci ne puise se faire que dans le sens désiré (figures 5g et 5h).

le génome du plasmide pBR322 est ainsi reconstitué sans modification ni délétion dans le plasmide recombinant. En particulier, les régions nécessaires à sa réplication et à l'expréssion de la résistance à la tétracycline ne sont pas touchées.

4. Transformation de la souche E. coli 1106

Les fragments des plasmides pPVI-846 et −120 recollés par leurs sites Kpn I et EcoRI sont mis en contact avec des bactéries compétentes de souche E. coli 1106 dans les conditions de transformation. Les colonies de bactéries résistantes à la tétracycline et sensibles à l'ampicilline sont sélectionnées.

5. Analyse des nouveaux clones

5.1 le DNA plasmidique des bactéries tétracycline résistantes est purifié. Sa masse est déterminée par électrophorèse en gel d'agarose. Elle est égale à celle du plasmide pBR322 augmentée des 5650 paires de bases du cDNA viral formé par recombinaison.

5.2 L'hybridation in vitro des cDNA ainsi obtenus avec des sondes spécifiques provenant des clones pPVI-846 et pPVI-120 permet la vérification de la présence dans un seul clone recombinant du matériel génétique du poliovirus inséré originellement dans les deux clones parentaux.

5.3 L'analyse détaillée des nouveaux clones est effectuée par les méthodes utilisées antérieurement pour l'étude des clones déjà caractérisés (cartographie physique par enzymes de restriction, microscopie électronique, séquence nucléotidique, etc.).

5.4 Le cDNA porté par le plasmide recombinant (pPV1-X) ou pPV1-958 porte l'information génétique nécessaire à la synthèse de la protéine NCVP1a (ou P1), précurseur des protéines de capside VP4 (nucléotides 743 à 950), VP2 (nucléotides 951 à 1766), VP3 (1767 à 2479) et VP1 (2480 à 3385), suivie de celles qui correspondent à la protéine NCVP3b (ou P2) (précursur notamment de la protéine NCVPX) et au début de la protéine NCVP1b (ou P3) L'ensemble couvre environ 5650 des 7440 bases du génome viral.

Le plasmide pPVI-846 a été déposé à la C.N.C.M. sous le no I-155 et le plasmide 120 sous le no I-156 le 19 mai 1981.

Le plasmide pPVI-948 obtenu contient dans son insérat la séquence nucléotidique qui code pour les protéines VPO (nucléotides 743 à 1766), VP3 (nucléotides 1767 à 2479) et PVI (nucléotides 2480 à 3385) suivie de la séquence codant pour la protéine NCVP3b (nucléotides 3386 à 5100 et quelque) et du début de celle de la protéine NCVP1b.

Partant du plasmide pPV1-958, on peut alors obtenir un fragment de cDNA codant pour VP1 en procédant comme suit.

Isolement et reclonage d'un fragment de cDNA renfermant la sequence de VP1

La séquence de nucléotides qui code pour la protéine VP1 est encadrée, dans le génome viral, et par conséquent aussi dans l'insérat porté par pPV1-958, de deux sites PstI, situés respectivement 237 nucléotides en amont (position 2243) et 32 nucléotides en aval (position 3417) du premier et du dernier nucléotide de cette séquence (cf. carte de restriction dans la susdite publication et fig. 1 et 2).

Le découpage de pPV1-958 (fig. 6a) par l'enzyme de restriction PstI engendre donc une famille de fragments ayant des longueurs correspondant respectivement à 4,36 kb (corps du plasmide) et à 1,8 kb; 0,43 kb; 1,17 kb et environ 2,23 kb. Le fragment de 1,17 kb porte la séquence nucléotidique codant pour la fin de VP3 et la totalité de VPI. Ce dernier fragment commence par la séquence nucléotidique: $_{5'}$ G T C C T C A T G T A et s'achève par la séquence G T A C A C T G C A$_{3'}$. On le sépare des autres fragments PstI par électrophorèse en gel d'agarose. On prélève la bande du gel qui le contient, et on la soumet à une électroélution pour en extraire le DNA. On suit l'électroélution par illumination aux ultraviolets après coloration du gel au bromure d'éthidium. Le fragment ainsi préparé correspond aux nucléotides du poliovirus 2243 à 3417. On l'insère par ligation avec de la DNA-ligase au site PstI du plasmide vecteur pBR-322 préalablement linéarisé par cette même enzyme. Les plasmides recombinants qu'on a formés ainsi sont clonés dans la souche 1106 d'Escherichia coli (sélection des colonies devenues résistantes à la tétracycline mais demeurées sensibles à l'ampicilline après transformation par le plasmide).

L'analyse de leur ADN par cartographie aux enzymes de restriction permet d'identifier et de sélectionner les plasmides recombinants qui portent le fragment du cADN polioviral inséré dans le sens contraire aux aiguilles d'une montre par rapport à la carte de pBR-322, c'est-à-dire dans le même sens transcriptionnel que le gène de la β-lactamase (gène de la résistance à l'ampicilline). Il faut noter que l'insertion du fragment 2243-3417 au site PstI de pBR-322 interrompt la continuité de la séquence nucléotidique, et inactive donc le gène de la β-lactamase du vecteur, mais ne permet cependant pas d'assurer l'expression des protéines poliovirales car elle entraîne un changement de phase de lecture de l'insérat.

On dénomme pSW-11 (fig. 6b), le plasmide ayant ces propriétés.

Elimination des sequences codant pour la partie C terminale de VP3: emondage de VP1

Le plasmide pSW-11 contient, précédent, dans le sens transcriptionnel 5 → 3', la séquence de VP1, 237 nucléotides de cADN de poliovirus relevant de la séquence de VP3. Ces nucléotides excédentaires peuvent être enlevés d'au moins deux manières:

a) par traitement ménagé du fragment Pstl (préalablement extrait de pSW-11: fig. 6c) de 1,17 kb par l'enzyme de restriction Haell (digestion partielle au nucléotide 2467), puis sélection par électrophorèse du fragment Haell-Pstl de 0,95 kb (fig. 6d) (nucléotides polioviraux 2467 à 3417) et reclonage de ce fragment dans les plasmides appropriés. On peut faciliter le reclonage en accrochant de façon en soi connue aux extrémités du fragment émondé des adaptateurs ("linkers") synthétiques, courtes séquences de nucléotides contenant un site de restriction déterminé obtenu par synthèse, par exemple selon la technique décrite par R. H. SCHELLER et al, Science, tome 196 (1977), pp. 177—180. Le type de "linker" choisi dépend essentiellement du site de coupure de l'enzyme de restriction utilisée dans le vecteur d'expression.

b) par linéarisation du plasmide pSW-11 par digestion complète par l'enzyme Pvul, suivie d'un traitement exonucléolytique à l'enzyme Bal 31, puis de l'addition éventuelle d'adaptateurs ("linkers") synthétiques types (Biolabs, Collaborative Research) et de la recircularisation du plasmide par la DNA ligase.

On ouvre donc les molécules. On analyse par migration en électrophorèse en gel d'agarose leurs tailles pour identifier celles qui ont perdu environ 700 paires de bases (perte qui dans la figure 6e est symbolisée par un arc de cercle en pointillés), c'est-à-dire quelque 350 de part et d'autre du site Pvul, soit le fragment Pvul-Pstl de pBR-322 plus la séquence de VP3 jusqu'à VP1, d'un côté, et en une longueur similaire de pBR-322 allant de Pvul vers EcoRl, l'autre côté.

De cette façon, on peut isoler un fragment dont l'une des extrémités coïncide avec l'extrémité de la séquence d'ADN codant pour VP1; ou en tous les cas en est très proche.

En effet, le site Pvul se trouvait à 126 paires de bases (b) du site proximal Pstl de la séquence du fragment Pstl de 1,17 kb et à 363 paires de bases de l'extrémité proximale du fragment de cDNA codant pour VP1, dans le plasmide pSW-11.

Après fixation éventuelle aux extrémités du fragment sélectionné de "linkers" contenant par exemple un site Bg/II au moyen d'une ligase, on sélectionne les plasmides dont la taille est de 4,8 à 5 kb (fig. 6f). On détermine ensuite ceux des plasmides qui ont conservé la séquence entière de VP1, tout en ayant perdu la totalité ou la quasi-totalité de VP3. On vérifie cela dans le cas où l'on a inséré un linker Bg/II en déterminant la séquence nucléotidique du fragment Bg/II-Pstl des plasmides sélectionnés. Si l'on utilise la méthode de SANGER, on insère les fragments à séquencer dans la forme réplicative du phase M13 et on clone les phages recombinants ainsi constitués. On utilise ensuite leur ADN pour séquencer le fragment inséré, selon la technique décrite par SANGER. On peut aussi procéder à la détermination de la séquence nucléotidique conservée par la méthode décrite par MAXAM et GILBERT.

Le plasmide obtenu par émondage du plasmide pSW-11, notamment par la variante b du procédé décrit ci-dessus, mais sans introduction de linker Bg/II, a été dénommé pSW-119.

Les différences observées entre les plasmides pSW-11 et pSW-119 (ou pCW-119) résultent du schéma de la fig. 7. En pariculier, le plasmide pSW-119 a perdu la plus grande partie de la partie de séquence que contenait encore le plasmide pSW-11 et qui code pour le polypeptide de structure VP3 du poliovirus.

Comme cela à été indiqué dans la demande de brevet français n° 82 02013, le plasmide pSW-119 est capable d'exprimer une protéine de fusion VP1-β-lactamase dans des souches de E. coli 1106 ou GC 26 (entre autres micro-organismes, tels que ceux envisagés dans la demande de brevet antérieur n° 82 02013). Cette protéine de fusion, ayant un poids moléculaire de 49.000 daltons, est spécifiquement immunoprécipitée par les anticorps monoclonaux CD-VP1 (ou C3).

Un dérivé de pSW 119, pFS 119, a été construit en remplaçant les séquences entre les sites BamHl et Pstl de pBR 322 (nucléotides 375—3 608) par les séquences correspondantes de pBR 327. Après marquage des protéines exprimées par le plasmide pFS 119 dans des bactéries GC 26 avec la ($^{35}$S)méthionine, immunoprécipitation et analyse par électrophorèse sur gel de polyacrylamide, il a de nouveau été possible de reconnaître une protéine de fusion ayant un poids moléculaire de l'ordre de 49 000 daltons (p49), spécifiquement immunoprécipitée par C3, parmi les produits d'expression dans GC 26.

On a également représenté dans la fig 7 la structure schématique du plasmide pFS-1019, tel qu'il a été obtenu par:
— digestion du plasmide pSW-119 ou pFS-119
— séparation par électrophorèse des fragments obtenus en gel d'agarose,
— sélection (par la taille des fragments concernés) de celui des fragments dérivés de pSW-119 ou pCW-119 et présentant la taille de celui-ci, cependant réduite d'environ 315 paires de bases. On a de même recueilli le petit fragment de 315 paires de bases XBal-Xbal, également obtenu à partir du milieu de digestion dans les mêmes conditions.

Le premier fragment sélectionné a été recircularisé au moyen d'une ligase, pour former le plasmide pFS 1019. Après incorporation de celui-ci dans E. coli 1106, celle-ci a conduit à l'obtention d'une protéine de fusion tronquée, de 39.000 daltons, qui n'est plus reconnue par l'anticorps monoclonal CD-VP1 ou C3.

Au contraire, le petit fragment a extrémité Xhal de 315 nucléotides, conduit, lorsqu'il est réintroduit en phase dans un gène porté par un plasmide approprié, à un plasmide modifié capable de transformer *E. coli* 1106 pour rendre celle-ci capable d'exprimer une protéine hybride reconnue par les anticorps monoclonaux C3. Par exemple, cette réintroduction en phase peut être effectuée dans le gène de β-lactamase de pBR-322.

La mise en phase adéquate peut, si besoin, être réalisée par la technique décrite dans la demande de brevet français n° 78 32041 du 13 novembre 1978.

La réinsertion du fragment *Xba*I-*Xba*I, quel que soit son origine, dans le plasmide pFS 1019 conduit à nouveau à une plasmide dont les produits d'expression contiennent une protéine reconnaissable par C3. Il en a particulièrement été ainsi en ce qui concerne le plasmide pCW 119, lequel à été obtenu par réinsertion dans le site *Xba*I de pFS 1019 d'un fragment *Xba*I-*Xba*I de même taille et structure nucléotidique, isolé à partir de pPV1-366 également décrit dans la demande de brevet 81 09 968.

La séquence nucléotidique du fragment Xbal-Xbal (315 paires de nucléotides) a déjà été indiquée plus haut. De même à été indiquée plus haut la séquence peptidique du peptide Ser 23—Ser 128.

On a représenté dans la figure 9 un schéma du plasmide pCW 119, dans lequel la séquence codant pour la protéine VP 1 a été représentée par une zone hachurée délimitée par deux arcs de cercles. Les principaux sites considérés dans le cadre de la présente description sont également indiqués dans la figure 9.

La détermination et l'obtention de séquences peptidiques plus petites susceptibles de porter le site immunogène recherché (ou épitope reconnu par C3) ont été conduites de la façon suivante.

Le plasmide pCW 119 a été soumis à une digestion par l'enzyme de restriction Kpn I, ce qui l'a ouvert au niveau du site de restriction 3064 (par conséquent extérieur au fragment *Xba*I susdit). La mise en oeuvre du procédé défini ci-dessus, mettant en jeu des cycles répétés d'émondage avec l'enzyme *Bal* 31, a conduit à la perte de fragments successifs d'extrémités, dont ceux codant pour les susdites séquences "His 65—Phe 105" et "Asp 93—Leu 104". La délétion à partir des plasmides linéarisés des fragments contenant les séquences codant pour les séquences peptidiques qui viennent d'être mentionnées, s'est manifestée par la perte chez le plasmide postérieurement recircularisé (au moyen de T4-ADN-ligase) de sa capacité d'induire la production, par les bactéries transformées par lui, de séquences peptidiques susceptibles d'être reconnues par les anticorps monoclonaux CD-PV1 ou C3.

Afin de localiser avec davantage encore de précision l'épitope reconnu par C3, on a construit une série de plasmides dérivés de pCW 119 et comportant des délétions plus ou moins étendues de cette séquence. Les positions relatives des fragments délétés vis-à-vis des sites *Xba*I (2546) et (2 861) sont schématisées par les arcs de cercles apparaissant dans la figure 9. Les limites de ces délétions vers la gauche ont été déterminées par linéarisation des plasmides (à raison de 1 µg d'ADN) avec *Xba*I et par marquage avec l'enzyme de Klenow pendant une heure à 15°C, en présence de [α$^{32}$p]-dATP (10 µCi) et de dGTP, dCTP et dTTP (à raison de 0,2 mM de chacun de ces derniers constituants). L'ADN marqué a ensuite été digéré au moyen des enzymes de restriction indiqués ci-après (conditions de digestion partielle lorsque l'on a recours à *Alu*I). Les fragments de restriction marqués ont ensuite été séparés sur un gel de polyacrylamide à 5% et rendus visibles par autoradiographie. Les limites des délétions vers la droite ont été déduites de la taille des fragments délétés et confirmées par la présence ou l'absence des sites de restriction pour les enzymes identifiés dans la partie supérieure de la fig. 10. Les symboles utilisés dans cette dernière avaient la signification suivante: X = *Xba*I; H = *Hha*I; A = *Alu*I; S = *Sau*3A; K = *Kpn*I; P = *Pst*I. Les numéros indiqués correspondent aux positions des nucléotides concernés vis-à-vis de la fig. 8.

Les protéines de fusion tronquées exprimées par les plasmides pCW217, 213, 215, et 202 réagissent encore avec l'anticorps monoclonal neutralisant C3. En revanche, les protéines de fusion tronquées exprimées par les plasmides pCW216, 203, 318 et 223 ne sont plus reconnues par l'anticorps C3. La cartographie fine par enzymes de restriction a permis de déterminer que la plus grande délétion n'affectant pas la réactivité de la protéine tronquée avec C3 (pCW215) s'étend jusqu'au nucléotide 2792

(Leu)
104

et que la plus petite délétion se traduisant par une perte de réactivité de la protéine tronquée s'étend jusqu'aux nucléotides 2771—2782

(Thr-Lys)
98  108

Par conséquent, il peut être considéré que l'extrémité C-terminale de la séquence d'acides aminés constituant l'épitope neutralisant reconnu par C3 est localisée entre les acides aminés 95, 110, et plus particulièrement encore les acides aminés 98 et 104 de la protéine VP1. Cette région correspond par ailleurs à une zone hydrophile de la protéine.

## Insertion de ces sequences d'ADN dans un vecteur d'expression

La séquence *Xba*I—*Xba*I ne comporte ni codon d'initiation, ni codon de terminaison. Elle ne comporte non plus ni promoteur pour sa transcription, ni signal de reconnaissance par les ribosomes (séquences de SHINE et DALGARNO, décrite dans GIRARD et HIRTH, Virologie Moléculaire, Edition Doin 1980, pp. 15—46 et 263—264). Pour la faire exprimer, il faut donc l'insérer en phase au milieu de la séquence nucléotidique 'et en tout cas derrière l'AUG d'initiation) d'un gène cloné avec son promoteur (ou auquel on adjoindra en amont, un promoteur étranger). L'utilisation de "linkers", comme décrit ci-dessus, permet d'envisager l'utilisation de plusieurs types de vecteurs d'expression différents selon le promoteur considéré: par exemple du type de ceux indiqués ci-après à titre d'exemple.

a) Promoteurs bactériens

C'est notamment le cas des plasmides contenant la région promoteur-opérateur de l'opéron lactose d'*E coli* (péron lac), suivie de la partie 5' du gène de la β-galactosidase. Ces vecteurs, du type pPC (CHARNAY et al, Nucleic Acid Research 1978, tome V, pp. 4 479—4494), permettent l'insertion de la séquence au site EcoRI situé à 21 nucléotides derrière l'AUG d'initiation de la β-galactosidase. La protéine à laquelle ils donnent naissance comporte donc pour extrémité N terminale les sept (ou huit) premiers acides aminés de la β-galactosidase bactérienne, suivis des acides aminés codés par ladite séquence.

b) Promoteurs de phage

C'est notamment le cas des plasmides contenant la région promoteur-opérateur de l'opéron de gauche ($P_L$) ou de l'opéron de droite ($P_R$) du phageλ. Ces vecteurs, respectivement du type pKC30 (ROSENBERG. Nature 1981, vol. 292, p. 128) ou pCL47 (ZABEAU et STANLEY, The EMBO Journal, 1982, tome I, pp. 1217—1224) dérivés du pLK5 (ou pRC5) et de pLG400, ce dernier étant décrit dans Cell, 1980, vol. 20, pp. 543—553) permettent d'effectuer l'insertion de ladite séquence au sein des séquences nucléotidiques codant respectivement pour l'extrémité N-terminale du produit du gène *N* ou pour celle du produit du gène *cro* déposé le 8 février 1982 à la C.N.C.M. sous le n° I-184. Ces systèmes de vecteurs sont propagés à 30°C dans des bactéries lysogénisées par un phageλ à répresseur thermosensible (cl 857) ou en présence de plasmides porteurs du même gène (*cl* 857) codant pour un répresseur thermosensible. Ils demeurent inactifs, du fait du répresseur, tant que la culture est maintenue à 30°C. Le transfert de la culture à 42°C est suivi de l'activation des promoteurs de λ ($P_L$ ou $P_R$) portés par le plasmide recombinant, du fait de l'inactivation du répresseur du gène *cl* 857.

c) Promoteurs viraux

C'est en particulier le cas de l'utilisation du virus SV40 comme vecteur. Dans ce cas, on utilise le promoteur viral tardif et l'on insère la séquence du poliovirus à la place de tout ou partie de la région codant pour les protéines tardives de SV40 (VP1 ou VP2). On construit ainsi des ADN de SV40 substitués dans lesquels les séquences codant pour les protéines de capside de ce virus sont remplacées par la séquence codant pour le peptide immunogène. Ainsi l'insertion de ladite séquence, au besoin par l'intermédiaire de "linkers" appropriés à la place du fragment tardif HaeII-PstI de SV40 (nucléotides de 767 à 1923), ou d'une partie de ce fragment, aboutit à la création d'un gène chimérique possédant une séquence codant pour un peptide immunogène inducteur *in vivo* d'anticorps actifs à l'égard du poliovirus directement en aval de la portion N-terminale de la protéine VP2 du SV40.

On peut procéder de même en substituant la séquence VP1 du poliovirus à celle du SV40 entre les sites EcoRI (1718) et BamH1 (2469).

d) Promoteurs de virus animaux portés par des plasmides bactériens

C'est le cas des plasmides portant les promoteurs du gène de la thymidine-kinase du virus de l'herpès (pAGO), du gène de l'antigène HBs du virus de l'hépatite B (pAC-2 ou pAC-14 ou des gènes précoces ou tardifs de l'adénovirus 2 etc.. L'insertion d'une séquence immunogène selon l'invention derrière l'AUG du gène viral cloné avec son promoteur permet d'en assurer l'expression dans la cellule animale (après transfection, micro-injection ou fusion cellules-protoplastes).

Les séquences peptidiques ou "séquences selon l'invention" sont accessibles par synthèse chimique, par exemple en ayant recours à l'un des procédés classiques dont les conditions de mise en oeuvre sont rappelées ci-après.

La synthèse des peptides en solution homogène et en phase solide est bien connue.

A cet égard, on pourra avoir recours au mode de synthèse en solution homogène décrit par HOUBENWEYL dans l'ouvrage intitulé "Methodem der Organischen Chemie" (Méthode de la chimie organique) édité par E. Wünsch., vol. 15—I et II, THIEME, Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthylaminopropyl)-carbodiimide. Lorsque l'aminoacyle mis en oeuvre possède une fonction amine supplémentaire (cas de la lysine par exemple) ou une autre fonction acide (cas par exemple de l'acide

9

glutamique), ces fonctions seront par exemple protégées, par des groupes carbobenzoxy ou t-butyloxycarbonyle, en ce qui concerne les fonctions amine, ou par des groupes t-butylester, en ce qui concerne les fonctions carboxyliques. Il en ira de même de la protection de toute autre fonction réactive. Par exemple, lorsque l'un des aminoacyles considérés contient une fonction SH (par exemple le cystéine), on pourra avoir recours à un groupe acétamidométhyle ou paraméthoxybenzyle.

Dans le cas de la synthèse progressive, acide aminé par acide aminé, la synthèse débute de préférence par la condensation de l'amino-acide C-terminal avec l'amino-acide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R. D. MERRIFIELD dans l'article institué "Solid phase peptide synthesis" (J. Am. Chem. Soc., 45, 2149—2154).

Pour fabriquer une chaîne peptidique selon le procédé de MERRIFIELD, on a recours à une résine polymère très poreuse, sur laquelle on fixe le premier amino-acide C-terminal de la chaîne. Cet amino-acide est fixé sur la résine par l'intermédiaire de son groupe carboxylique et sa fonction amine est protégée, par exemple par le groupe t-butyloxycarbonyle.

Lorsque le premier amino-acide C-terminal est ainsi fixé sur la résine, on enlève le groupe protecteur de la fonction amine en lavant la résine avec une acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième amino-acide qui fournit le second amino-acyle de la séquence recherchée, à partir du résidu amino-acyle C-terminal sur la fonction amine déprotégée du premier amino-acide C-terminal fixé sur la chaîne. De préférence, la fonction carboxyle de ce deuxième amino-acide est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux aminoacides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés, qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

Detection de l'expression des sequences immunogenes selon l'invention

L'expression des plasmides recombinants porteurs desdites séquences immunogènes et capables de les exprimer, c'est-à-dire d'effectuer la synthèse d'un peptide immunogène, est détectée par des techniques d'immunoprécipitation, en elles-mêmes connues et mettant de préférence en jeu des liquides d'ascite contenant les anticorps monoclonaux C3 ou sérum de lapin anti-VP1 (α VP1).

En ce qui concerne les séquences de plus petite taille et portant un épitope ou déterminant immunogénique, et plus particulièrement celles qui sont accessibles de façon relativement aisée par synthèse chimique, il sera souhaitable, en vue d'accentuer leur caractère immunogène *in vivo*, de les coupler ou "conjuguer" de façon covalente à une molécule porteuse, physiologiquement acceptable et non toxique.

A titre d'exemples de molécules porteuses ou supports macromoléculaires entrant dans la constitution des conjugués selon l'invention, on mentionnera des protéines naturelles, telles que la toxine tétanique, l'ovalbumine, des sérums albumines, des hémocyanines, etc.

A titre de supports macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20.000.

Pour synthétiser les conjugués selon l'invention, on peut avoir recours à des procédés connus en soi, tel que celui décrit par FRANTZ et ROBERTSON dans Infect. and Immunity, *33*, 193—198 (1981), ou celui décrit dans Applied and Environmental Microbiology, octobre 1981, vol. 42, n° 4, 611—614 par P. E. KAUFFMAN en utilisant le peptide et la molécule porteuse appropriée.

Dans la pratique, on utilisera avantageusement comme agent de couplage les composés suivants, cités à titre non limitatif: aldéhyde glutarique, chloroformiate d'éthyle, carbodiimides hydrosolubles [N-éthyl-N'(3-diméthylamino-propyl) carbodiimide, HCl], diisocyanates, bis-diazobenzidine, di- et trichloro-s-triazines, bromures de cyanogène, benzaquinone, ainsi que les agents de couplage mentionnés dans Scand. J. Immunol., 1978, vol. 8, p. 7—23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminopropyl)-carbodiimide, le N-hydroxybenzotriazole, etc. On peut encore avoir recours à la glutaraldéhyde, notamment lorsqu'il s'agit de relier entre eux des groupes aminés respectivement portés par le peptide et la molécule support.

On mentionne ci-après à titre d'exemple le couplage du peptide Asp 93—Leu 104 à une molécule support constituée par l'hèmocyanine(KLH) de l'anglais "Keyhole limpet hemocyanin" au moyen de glutaraldéhyde selon la méthode décrite par BOQUET, P. et Coll. (1982) Molec. Immunol., 19, 1541—1549. Le couplage est fait à partir de proportions d'environ 2 mg de peptide pour 2,25 mg de l'hémocyanine.

Le conjugué obtenu est immunoprécipitable par des anticorps monoclonaux C3.. Cette immunoprécipitation peut être suivie par marquage du conjugué avec $^{125}I$ en utilisant de la chloramine T. Etant donné que le peptide ne contient pas de résidus tyrosine, le marquage n'intervient qu'au niveau de la protéine support, de sorte que les propriétés antigèniques du peptide n'ont pas pu être modifiées.

L'immunogènicité de ces peptides peut également être renforcée, en produisant leur oligomérisation, par exemple en présence de glutaraldéhyde ou tout agent permettant la mise en jeu du couplage de fonctions réactives distinctes portées par chacun des peptides monomères; en particulier, l'invention concerne les oligomères immunogènes hydrosolubles ainsi obtenus, comprenant notamment de 2 à 10 motifs monomères.

D'une façon générale, l'invention concerne tous petits "peptides immunogènes" contenant moins de 20 résidus aminoacyle, de préférence moins de 15 résidus aminoacyle. Ces peptides immunogènes contiennent de préférence la séquence Asp 93—Leu 104 sus-indiquée ou toute séquence ayant une structure conformationnelle semblable.

L'invention ne se limite naturellement pas aux peptides particuliers qui ont été envisagés.

Comme il est bien connu de l'homme de l'art, certains résidus aminoacyle contenus dans les séquences considérées peuvent éventuellement être remplacés par d'autres résidus aminoacyle, dans la mesure où ceux-ci ne modifient pas sensiblement les configurations de surface des peptides formés, et leur aptitude, notamment après leur couplage avec le support macromoléculaire, à réagir avec les anticorps dirigés contre les poliovirus. A cet égard, on mentionnera par exemple les éventuelles substitutions du groupe alanyle par le groupe glycyle ou vice-versa, la substitution possible des résidus iso-asparagiques par des résidus aspartiques, glutamine ou isoglutamine, la substitution des groupes valine par les groupes alanine, leucine ou glycine, la substitution des groupes lysine par des groupes norleucine ou encore arginine, etc., sous réserve que soit chaque fois vérifiée la capacité des peptides modifiés d'induire des anticorps susceptibles de neutraliser le poliovirus entier ou d'être reconnus par les anticorps monoclonaux CD-VP1. Il est naturellement entendu que tous ces équivalents possibles entrent dans le champ des revendications ci-après.

### Proprietes des peptides selon l'invention

Les peptides selon l'invention, et plus particulièrement les peptides conjugués formés, sont capables d'induire *in vivo* la production d'anticorps selon les techniques classiques. On peut les faire réagir avec les anticorps antipoliovirus. Ils induisent la synthèse d'anticorps antipoliovirus, lorsqu'ils sont inoculés à l'animal.

Par ailleurs on peut les utiliser comme réactifs pour le diagnostic et le titrage des anticorps antipoliomyélitiques. Dans leur utilisation comme réactifs pour un diagnostic, on peut avoir recours à des techniques classiques, par exemple à la technique ELISA. On rappelle ci-après le principe d'une telle méthode. Elle comprend, par exemple, les étapes suivantes:

— dépôt de quantités déterminées du peptide selon l'invention dans les puits d'une microplaque du type de celle utilisée pour la mise en oeuvre de la méthode ELISA:

— introduction de dilutions croissantes du sérum contenant, le cas échéant, les anticorps à détecter, voire à doser, dans les puits de cette microplaque;

— incubation et interruption de la réaction, par exemple par addition d'une solution d'acide sulfurique;

— lavage poussé de la microplaque avec un tampon approprié;

— introduction d'anticorps marqués dirigés contre les premiers, le marquage étant fait au moyen d'une enzyme capable d'hydrolyser un substrat choisi parmi ceux pour lesquels cette hydrolyse se manifeste par une variation d'absorbance pour une radiation de longueur d'onde donnée,

— mesure de la variation d'absorbance et

— détermination, de préférence vis-à-vis de mesures semblables faites vis-à-vis d'un témoin, de la teneur en anticorps du sérum étudié.

Les séquences d'ADN selon l'invention peuvent elles-mêmes être utilisées comme sondes d'hybridation permettant la détection de la présence d'ARN viral ou du cADN correspondant dans un échantillon biologique. Cette méthode impliquera par conséquence l'extraction préalable de l'ARN ou ADN de l'échantillon biologique et sa mise en contact dans des conditions permettant l'hybridation avec la séquence d'ADN selon l'invention marquée par un traceur radio-actif ou par une enzyme, notamment du genre de celles qui sont aptes à hydrolyser un substrat du type us-indiqué.

L'invention concerne naturellement toutes les séquences d'ADN équivalentes conduisant à des produits d'expression doués de propriétés immunologiques équivalentes, en ce que les anticorps induits par les produits d'expression de ces séquences équivalentes sont à même de réagir avec les produits d'expression des fragments d'ADN plus particulièrement décrits et vice versa. En particulier, l'invention s'étend à des séquences d'ADN pouvant différer de celles qui ont été plus particulièrement décrites, par des délétions, additions ou substitutions d'acides nucléiques, pour autant que les propriétés immunologiques des produits d'expression soient équivalentes.

L'invention concerne encore en procédé d'obtention d'un peptide immunogène tel que ci-dessus défini comprenant les étapes que sont l'insertion d'une séquence d'ADN conforme à l'invention dans un vecteur approprié, la transformation d'un micro-organisme transformable par le vecteur ainsi modifié et capable d'exprimer la susdite séquence d'insertion, la récupération des protéines synthétisées et l'isolement de la fraction peptidique contenant le peptide selon l'invention, celui-ci pouvant être détecté, les cas échéant après un frationnement en fonction des poids moléculaires, par des anticorps actifs à la fois contre les particules "C" et "D" d'un même poliovirus et contre le poliopeptide structural VP-1 de la capside de ce poliovirus.

L'invention concerne naturellement également tout vecteur contenant une séquence d'insertion conforme à l'invention, sous le contrôle d'un promoteur permettant l'expression de cet insérat dans un micro-organisme transformable par ce vecteur.

Enfin l'invention concerne les micro-organismes transformés par un tel vecteur, aptes à produire une protéine reconnue par des anticorps actifs à la fois contre les particules "C" et "D" d'un même poliovirus et contre le polypeptide structural VP-1 de la capside de ce poliovirus.

**Revendications pour les Etats contractants: BE CH DE FR GB IT LI LU NL SE**

1. Fragment d'ADN contenant une séquence d'au plus 318 paires de nucléotides, caractérisé en ce qu'il code pour un peptide contenant tout ou partie de la séquence d'aminoacides:

```
         Ser Arg Asp Ala Leu Pro Asn Thr Glu
     Ala Ser Gly Pro Thr His Ser Lys Glu Ile
     Pro Ala Leu Thr Ala Val Glu Thr Gly Ala
     Thr Asn Pro Leu Val Pro Ser Asp Thr Val
     Gln Thr Arg His Val Val Gln His Arg Ser
     Arg Ser Glu Ser Ser Ile Glu Ser Phe Phe
     Ala Arg Gly Ala Cys Val Thr Ile Met Thr
     Val Asp Asn Pro Ala Ser Thr Thr Asn Lys
     Asp Lys Leu Phe Ala Val Trp Lys Ile Thr
     Tyr Lys Asp Thr Val Gln Leu Arg Arg Lys
     Leu Glu Phe Phe Thr Tyr Ser
```

étant entendu qu'elle contient en tous les cas la séquence d'aminoacides:

Asp Asn Pro Ala Ser Thr Thr Asn Lys Asp Lys Leu.

2. Fragment d'ADN selon la revendication 1, caractérisé en ce que la séquence de 318 paires de nucléotides est caractérisée par la structure:

```
         TCT AGA GAC GCT CTC CCA AAC ACT GAA
     GCC AGT GGA CCA ACA CAC TCC AAG GAA ATT
     CCG GCA CTC ACC GCA GTG GAA ACT GGG GCC
     ACA AAT CCA CTA GTC CCT TCT GAT ACA GTG
     CAA ACC AGA CAT GTT GTA CAA CAT AGG TCA
     AGG TCA GAG TCT AGC ATA GAG TCT TTC TTC
     GCG CGG GGT GCA TGC GTG ACC ATT ATG ACC
     GTG GAT AAC CCA GCT TCC ACC ACG AAT AAG
```

GAT AAG CTA TTT GCA GTG TGG AAG ATC ACT

TAT AAA GAT ACT GTC CAG TTA CGG AGG AAA

TTG GAG TTC TTC ACC TAT TCT.

3. Fragment d'ADN selon la revendication 1 ou la revendication 2, caractérisé en ce qu'il contient la séquence nucléotidique codant pour la séquence peptidique His 65—Phe 105:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe

100

4. Fragment d'ADN selon la revendication 1 ou la revendication 2, caractérisé en ce qu'il contient la séquence nucléotidique:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe Ala Val Trp Lys

100

Ile

110

5. Fragment selon la revendication 1 ou 2, caractérisé en ce qu'il code pour une séquence:

Asp Asn Pro Ala Ser Thr Thr Asn Lys Asp Lys Leu.

6. Polypeptide contenant tout ou partie de la séquence d'aminoacides de formule:

Ser Arg Asp Ala Leu Pro Asn Thr Glu

Ala Ser Gly Pro Thr His Ser Lys Glu Ile

Pro Ala Leu Thr Ala Val Glu Thr Gly Ala

Thr Asn Pro Leu Val Pro Ser Asp Thr Val

Gln Thr Arg His Val Val Gln His Arg Ser

13

```
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser
```

ce polypeptide contenant en tous les cas la séquence:

Asp Asn Pro Ala Ser Thr Thr Asn Lys Asp Lys Leu.

7. Polypeptide selon la revendication 6, caractérisé en ce qu'il contient la séquence d'aminoacides de formule:

```
                        His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe
100
```

8. Polypeptide selon la revendication 6, caractérisé en ce qu'il contient la séquence d'aminoacides de formule:

```
                        His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe Ala Val Trp Lys
100
Ile
110
```

9. Polypeptide selon la revendication 6, caractérisé par la séquence d'acides aminés de formule:

Asp Asn Pro Ala Ser Thr Thr Asn Lys Asp Lys Leu

10. Polypeptide selon la revendication 6, caractérisé en ce qu'il contient moins de 20 résidus d'aminoacides.

11. Polypeptide selon la revendication 6, caractérisé en ce qu'il contient moins de 15 résidus d'aminoacides.

12. Conjugué immunogène résultant du couplage covalent d'un polypeptide selon l'une quelconque des revendications 6 à 11 avec une molécule porteuse, de préférence ayant un poids moléculaire supérieur à 20.000.

13. Vecteur contenant un insérat constitué par une séquence d'ADN placée sous le contrôle d'un promoteur permettant l'expression de cet insérat dans un micro-organisme transformable par ce vecteur, caractérisé en ce que la séquence de cet insérat nucléotidique est conforme à celle du fragment selon l'une quelconque des revendications 1 à 5.

14. Micro-organisme transformé par le vecteur selon la revendication 13 et apte à produire une protéine reconnue par des anticorps actifs à la fois contre les particules "C" et "D" d'un même poliovirus et contre le polypeptide structural VP-1 de la capside de ce poliovirus.

15. Oligomère hydrosoluble, contenant notamment de 2 à 10 motifs monomères, caractérisé en ce que l'unité monomère consiste en un polypeptide tel que défini dans l'une quelconque des revendications 6 à 11.

16. Procédé de préparation d'un peptide tel que défini dans l'une quelconque des revendications 6 à 11 comprenant la transformation d'un micro-organisme transformable par le vecteur selon la revendication 13 et dans lequel le susdit insérat peut être exprimé sous le contrôle du promoteur correspondant, la récupération des protéines synthétisées et l'isolement de la fraction peptidique contenant le susdit peptide, celui-ci pouvant être détecté, le cas échéant après un fractionnement en fonction des poids moléculaires, par des anticorps actifs à la fois contre les particules "C" et "D" d'un même poliovirus et contre le polipeptide structural VP-1 de la capside de ce poliovirus.

17. Réactif pour le diagnostic de la présence d'anticorps antipoliomyélitiques dans un échantillon biologique tel qu'un sérum, caractérisé en ce qu'il consiste en un antigène propre à réagir avec ces anticorps, cet antigène consistant en un polypeptide conforme à l'une quelconque des revendications 6 à 11 ou en un conjugué immunogène conforme à la revendication 12 ou encore en un oligomère immunogène conforme à la revendication 15.

18. Sonde pour la détection d'ARN du virus de la poliomyélite ou du cADN correspondant contenant une séquence nucléotidique capable de s'hybrider avec ledit ARN ou cADN, caractérisée en ce que cette sonde contient un fragment d'ADN conforme à l'une quelconque des revendications 1 à 5.

19. Utilisation d'un peptide selon l'une quelconque des revendications 1 à 5, d'un conjugué selon la revendication 12 ou d'un oligomère selon la revendication 15, pour la fabrication de principes de vaccins contre des poliovirus.

**Revendications pour l'Etat contractant: AT**

1. Procédé pour la synthèse d'un polypeptide contenant tout ou partie de la séquence d'aminoacides de formule:

```
Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser
```

ce polypeptide contenant en tous les cas la séquence:

Asp Asn Pro Ala Ser Thr Thr Asn Lys Asp Lys Leu,

caractérisé en ce que, partant de préférence de l'aminoacide C-terminal, l'on condense successivement deux à deux les aminoacyles successifs dans l'ordre requis, ou des aminoacyles et des fragments

# EP 0 110 791 B1

préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

2. Procédé selon la revendication 1, caractérisé en ce que l'on poursuit les condensations jusqu'à obtenir la séquence d'aminoacides de formule:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe

100

3. Procédé selon la revendication 1, caractérisé en ce que l'on poursuit la condensation jusqu'à obtenir la séquence d'aminoacides de formule:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe Ala Val Trp Lys

100

Ile

110

4. Procédé selon la revendication 1, caractérisé en ce que l'on poursuit les condensations jusqu'à obtenir la séquence d'acides aminés de formule:

Asp Asn Pro Ala Ser Thr Thr Asn Lys Asp Lys Leu.

5. Procédé selon la revendication 1, caractérisé en ce que l'on poursuit les condensations jusqu'à obtenir un polypeptide contenant moins de 20 résidus d'aminoacides.

6. Procédé selon la revendication 1, caractérisé en ce que l'on poursuit les condensations jusqu'à obtenir un polypeptide contenant moins de 15 résidus d'aminoacides.

7. Procédé pour la production d'un conjugué immunogène résultant du couplage covalent d'un polypeptide selon l'une quelconque des revendications 1 à 6 avec une molécule porteuse, de préférence ayant un poids moléculaire supérieur à 20.000, et comportant une ou plusieurs fonctions réactives carboxyle ou amine, caractérisé en ce que l'on fait réagir le peptide et la molécule support, en présence d'un agent de couplage du genre de ceux utilisés dans la synthèse des protéines, par example le N-hydroxybenzotriazole ou, en présence de glutaraldéhyde, notamment lorsqu'il s'agit de relier entre eux des

16

groupes aminés respectivement portés par le peptide et la molécule support.

8. Procédé de fabrication d'un oligomère hydrosoluble contenant notamment de 2 à 10 motifs monomères, dans lesquels l'unité monomère consiste en un polypeptide tel que défini dans l'une quelconque des revendications 1 à 6, caractérisé en ce que l'on fait réagir ces monomères en présence de glutaraldéhyde ou tout agent permettant la mise en jeu du couplage de fonctions réactives distinctes portées par chacun des polypeptides monomères.

9. Procédé de préparation d'un peptide tel que défini dans l'une quelconque des revendications 1 à 6 contenant un insérat formé par une séquence d'ADN codant pour l'un des polypeptides tels qu'obtenus par le procédé selon l'une quelconque des revendications 1 à 6, sous le contrôle d'un promoteur caractérisé par la transformation d'un micro-organisme transformable par le susdit vecteur et dans lequel le susdit insérat peut être exprimé sous le contrôle dudit promoteur, la récupération des protéines synthétisées et l'isolement de la fraction peptidique contenant le susdit peptide, celui-ci pouvant être détecté, le cas échéant après un fractionnement en fonction des poids moléculaires, par des anticorps actifs à la fois contre les particules "C" et "D" d'un même poliovirus et contre le poliopeptide structural VP-1 de la capside de ce poliovirus.

10. Procédé pour le diagnostic *in vitro* de la présence d'anticorps antipoliomyélitiques dans un échantillon biologique tel qu'un sérum, caractérisé en ce que l'on met en contact cet échantillon avec un polypeptide obtenu par le procédé conforme à l'une quelconque des revendications 1 à 6 ou 9 ou avec un conjugué immunogène obtenu par le procédé selon la revendication 7 ou avec l'oligomère hydrosoluble obtenu par le procédé selon la revendication 8.

**Patentansprüche für die Vertragsstaaten: BE CH DE FR GB IT LI LU NL SE**

1. DNA-Fragment, das eine Sequenz von höchstens 318 Nucleotidpaaren enthält, dadurch gekennzeichnet, daß es ein Peptid kodiert, das die gesamte nachfolgend genannte Aminosäuresequenz oder der einen Teil davon enthält:

```
        Ser Arg Asp Ala Leu Pro Asn Thr Glu
    Ala Ser Gly Pro Thr His Ser Lys Glu Ile
    Pro Ala Leu Thr Ala Val Glu Thr Gly Ala
    Thr Asn Pro Leu Val Pro Ser Asp Thr Val
    Gln Thr Arg His Val Val Gln His Arg Ser
    Arg Ser Glu Ser Ser Ile Glu Ser Phe Phe
    Ala Arg Gly Ala Cys Val Thr Ile Met Thr
    Val Asp Asn Pro Ala Ser Thr Thr Asn Lys
    Asp Lys Leu Phe Ala Val Trp Lys Ile Thr
    Tyr Lys Asp Thr Val Gln Leu Arg Arg Lys
    Leu Glu Phe Phe Thr Tyr Ser
```

mit der Maßgabe, daß sie auf jeden Fall die folgende Aminosäuresequenz enthält:

Asp Asn Pro Ala Ser Thr Thr Asn Lys Asp Lys Leu.

2. DNA-Fragment nach Anspruch 1, dadurch gekennzeichnet, daß die Sequenz mit 318 Nucleotidpaaren gekennzeichnet ist durch die Struktur:

```
        TCT AGA GAC GCT CTC CCA AAC ACT GAA
        GCC AGT GGA CCA ACA CAC TCC AAG GAA ATT
        CCG GCA CTC ACC GCA GTG GAA ACT GGG GCC
        ACA AAT CCA CTA GTC CCT TCT GAT ACA GTG
        CAA ACC AGA CAT GTT GTA CAA CAT AGG TCA
        AGG TCA GAG TCT AGC ATA GAG TCT TTC TTC
```

17

```
GCG CGG GGT GCA TGC GTG ACC ATT ATG ACC
GTG GAT AAC CCA GCT TCC ACC ACG AAT AAG
GAT AAG CTA TTT GCA GTG TGG AAG ATC ACT
TAT AAA GAT ACT GTC CAG TTA CGG AGG AAA
TTG GAG TTC TTC ACC TAT TCT.
```

3. DNA-Fragment nach Anspruch 1 oder 2, dadurch gekennzeichnet, daß es die Nucleotidsequenz enthält, welche die Peptidsequenz His 65-Phe 105 kodiert:

```
                            His Val Val Gln His
           Arg Ser Arg Ser Glu Ser Ser Ile Glu Ser
           70
           Phe Phe Ala Arg Gly Ala Cys Val Thr Ile
           80
           Met Thr Val Asp Asn Pro Ala Ser Thr Thr
           90
           Asn Lys Asp Lys Leu Phe
           100
```

4. DNA-Fragment nach Anspruch 1 oder 2, dadurch gekennzeichnet, daß es die Nucleotidsequenz enthält:

```
                            His Val Val Gln His
           Arg Ser Arg Ser Glu Ser Ser Ile Glu Ser
           70
           Phe Phe Ala Arg Gly Ala Cys Val Thr Ile
           80
           Met Thr Val Asp Asn Pro Ala Ser Thr Thr
           90
           Asn Lys Asp Lys Leu Phe Ala Val Trp Lys
           100
           Ile
           110
```

5. Fragment nach Anspruch 1 oder 2, dadurch gekennzeichnet, daß es eine Sequenz

Asp Asn Pro Ala Ser Thr Thr Asn Lys Asp Lys Leu

kodiert.

6. Polypeptid, das die gesamte Aminosäuresequenz oder nach folgenden Formel oder einen Teil davon enthält:

```
           Ser Arg Asp Ala Leu Pro Asn Thr Glu
           Ala Ser Gly Pro Thr His Ser Lys Glu Ile
           Pro Ala Leu Thr Ala Val Glu Thr Gly Ala
           Thr Asn Pro Leu Val Pro Ser Asp Thr Val
```

Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser,

wobei dieses Polypeptide auf jeden Fall die Sequenz:

Asp Asn Pro Ala Ser Thr Thr Asn Lys Asp Lys Leu

enthält.

7. Polypeptid nach Anspruch 6, dadurch gekennzeichnet, daß es folgende Aminosäuresequenz enthält:

His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe
100

8. DNA-Fragment nach Anspruch 6, dadurch gekennzeichnet, daß es die Aminosäuresequenz der folgenden Formel enthält:

His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe Ala Val Trp Lys
100
Ile
110

9. Polypeptid nach Anspruch 6, gekennzeichnet durch die Aminosäuresequenz der Formel:

Asp Asn Pro Ala Ser Thr Thr Asn Lys Asp Lys Leu.

10. Polypeptid nach Anspruch 6, dadurch gekennzeichnet, daß es weniger als 20 Aminosäurereste enthält.

11. Polypeptid nach Anspruch 6, dadurch gekennzeichnet, daß es weniger als 15 Aminosäurereste enthält.

12. Konjugiertes Immunogen, erhalten durch covalente Kupplung eines Polypeptids nach einem der Ansprüche 6 bis 11 mit einem Trägermolekül, vorzugsweise mit einem Molekulargewicht von über 20.000.

13. Vektor, der ein Insert enthält, daß durch eine DNA-Sequenz gebildet ist, die der Kontrolle eines Promotors unterworfen ist, welcher die Expression dieses Inserts in einem transformierbaren Mikroorganismus durch diesen Vektor ermöglicht, dadurch gekennzeichnet, daß die Sequenz dieses Nucleotidinserts derjenigen des Fragments nach einem der Ansprüche 1 bis 5 entspricht.

14. Mikroorganismus, der durch den Vektor nach Anspruch 13 transformiert und befähigt ist, ein durch Antikörper, die gleichzeitig gegenüber den Teilchen "C" und "D" ein und desselben Poliovirus und gegenüber dem Strukturpoliopeptid VP-1 des Kapsids dieses Poliovirus erkanntes Protein zu produzieren.

15. Wasserlösliches Oligomer, das 2 bis 10 Monomereinheiten enthält, dadurch gekennzeichnet, daß die Monomereinheit aus einem Polypeptid, wie in einem der Anprüche 6 bis 11 definiert, besteht.

16. Verfahren zur Herstellung eines Peptids wie in einem der Ansprüche 6 bis 11 definiert, durch Transformation eines Mikroorganismus, der durch den Vektor nach Anspruch 13 transformierbar ist und in dem das genannte Insert unter der Kontrolle des entsprechenden Promotors exprimiert werden kann, die Gewinnung der synthetisierten Proteine und die Isolierung der Peptidfranktion, welche das genannte Peptid enthält, wobei dieses nachgewiesen werden kann, gegebenenfalls nach einer Fraktionierung entsprechen dem Molekulargewicht, durch Antikörper, die gleichzeitig gegenüber den Teilen "C" und "D" ein und desselben Poliovirus und gegenüber dem Strukturpoliopeptid VP-1 des Kapsids dieses Poliovirus aktiv sind.

17. Reagens zum Nachweis der Anwesenheit von Antipoliomyelitisantikörpern in einer biologischen Probe wie in einem Serum, dadurch gekennzeichnet, daß es aus einem Antigen besteht, das zur Reaktion it diesen Antikörpern befähigt ist, wobei das Antigen aus einem Polypeptid nach einem der Ansprüche 6 bis 11 oder einem konjugierten Immunogen nach Anspruch 12 oder außerdem einem immunogenen Oligomer nach Anspruch 15 besteht.

18. Sonde zum Nachweis der RNA des Poliomyelitisvirus oder der entsprechenden cDNA, die eine Nucleotidsequenz enthält, die zur Hybridiserung mit dieser RNA oder cDNA befähigt ist, dadurch gekennzeichnet, daß diese Sonde ein DNA-Fragment nach einem der Ansprüche 1 bis 4 enthält.

19. Verwendung eines Peptids nach einem der Ansprüche 1 bis 5 eines Konjugats nach Anspruch 12 oder eines Oligomers nach Anspruch 15 zur herstellung von Impfstoffwirkstoffen gegen Polioviren.

**Patentansprüche für den Vertragsstaat: AT**

1. Verfahren zur Synthese eines Polypeptids, das die gesamte Aminosäuresequenz der nachfolgenden Formel oder einen Teil davon enthält;

```
Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser,
```

wobei dieses Polypeptid auf jeden Fall die Sequenz:

Asp Asn Pro Ala Ser Thr Thr Asn Lys Asp Lys Leu

enthält, dadurch gekennzeichnet, daß man vorzugsweise ausgehend von einer C-endständigen Aminosäure die aufeinanderfolgender Aminoacyle in der erforderlichen Reihenfolge paarweise oder Aminoacyle und vorgängig gebildete und bereits mehrere Aminoacylreste enthaltende Fragmente in der geeigneten Reihenfolge oder mehrere auf diese Weise vorgängig hergestellte Fragmente mit der Maßgabe, daß vorgängig alle reaktionsfähigen Gruppen, welche von diesen aminoacylen oder Fragmenten getragen werden, mit Ausnahme der Amingruppen des einen und der Carboxylgruppen des anderen oder vice versa, die normalerwise an der Bildung der Peptidbindungen teilnehmen müssen, insbesondere nach Aktivierung der Carboxylgruppe, geschützt wurden, nach in der Peptidsynthese bekannten Methoden usw. nach und nach biszur N-endständigen Aminosäure kondensiert.

2. Verfahren nach Anspruch 1, dadurch gekennzeichnet, daß man bis zur Erzielung der Aminosäuresequenz der nachfolgenden Formel weiterkondensiert:

```
                        His Val Val Gln His
Arg Ser Arg Ser Glu Ser Ser Ile Glu Ser
70

Phe Phe Ala Arg Gly Ala Cys Val Thr Ile
80

Met Thr Val Asp Asn Pro Ala Ser Thr Thr
90

Asn Lys Asp Lys Leu Phe
100
```

3. Verfahren nach Anspruch 1, dadurch gekennzeichnet, daß man bis zur Erzielung der Aminosdäuresequenz der nachfolgenden Formel weiterkondensiert:

```
                        His Val Val Gln His
Arg Ser Arg Ser Glu Ser Ser Ile Glu Ser
70

Phe Phe Ala Arg Gly Ala Cys Val Thr Ile
80

Met Thr Val Asp Asn Pro Ala Ser Thr Thr
90

Asn Lys Asp Lys Leu Phe Ala Val Trp Lys
100

Ile
110
```

4. Verfahren nach Anspruch 1, dadurch gekennzeichnet, daß man bis zur Erzielung der Aminosäuresequenz der nachfolgenden Formel weiterkondensiert:

Asp Asn Pro Ala Ser Thr Thr Asn Lys Asp Lys Leu.

5. Verfahren nach Anspruch 1, dadurch gekennzeichnet, daß man bis zur Erzielung eines Polypeptids mit wenigstens 20 Aminosäureresten weiterkondensiert.

6. Verfahren nach Anspruch 1, dadurch gekennzeichnet, daß man bis zur Erzielung eines Polypeptids mit wenigstens 15 Aminosäureresten weiterkondensiert.

7. Verfahren zur Herstellung eines konjugierten Immunogens durchkovalente Kupplung eines Polypeptids nach einem der Ansprüche 1 bis 6 mit einem Trägermolekül, vorzugsweise mit einem Molekulargewicht von über 20.000, wobei das Immunogen eine oder mehrere reaktionsfähige Carboxyl- oder Amingruppen aufweist, dadurch gekennzeichnet, daß man das Peptid mit dem Trägermolekül in Anwesenheit eines Kupplungsmittels in der Art, wie sie in der Proteinsynthese verwendet werden, wie z.B. in Anwesenheit von N-Hydroxybenztriazol oder Gutaraldehyd, insbesondere wenn es sich darum handelt, Amingruppen bzw. Gruppen, welche vom Peptid und dem Trägermolekül getragen werden, miteinander zu verknüpfen, umsetzt.

8. Verfahren zur Herstellung eines wasserlöslichen Oligomers, das 2 bis 10 Monomereinheiten enthält, wobei die Monomereinheit aus einem Polypeptid, wie in einem der Ansprüche 1 bis 6 definiert, besteht, dadurch gekennzeichnet, daß man diese Monomeren in Anwesenheit von Glutaraldehyd oder eines beliebigen Mittels, das die Kupplung der einzelnen reaktionsfähigen Gruppen an jedem der monomeren Polypeptide ermöglicht.

9. Verfahren zur herstellung eines Peptids, wie in einem der Ansprüche 1 bis 6 definiert, der ein Insert enthält, gebildet duch eine DNA-Sequenz, welche eines der Polypeptide kodiert, wie sich nach dem Verfahren nach einem der Ansprüche 1 bis 6 erhalten wurden, under der Kontrolle eines Promotors, und in welchem Mikroorganismus das genannte Insert unter der Kontrolle des Promotors exprimiert werden kann, die Gewinnung der synthetisierten Proteine und die Isolierung der Peptidfraktion, welche das genannte Peptid enthält, wobei dieses nachgewiesen werden kann, gegebenenfalls nach einer Fraktionierung entsprechend dem Molekulargewicht, durch Antikörper, die gleichzeitig gegenüber den Teilchen "C" und "D" ein und desselben Poliovirus und gegenüber dem Strukturpoliopeptid VP-1 des Kapsids dieses Poliovirus aktiv sind.

10. Verfahren zum Nachweis in vitro der Anwesenheit von Antiopoliomylitisantikörpern in einer biologischen Probe wie in einem Serum, dadurch gekennzeichnet, daß man diese Probe mit einem Polypeptid, erhalten nach dem Verfahren nach einem der Ansprüche 1 bis 6 oder 9, oder mit einem konjugierten Immunogen, erhalten nach dem Verfahren nach Anspruch 7, oder mit dem wasserlöslichen Oligomer, erhalten nach dem Verfahren nach Anspruch 8, in Berührung bringt.

**Claims for the Contracting States: BE CH DE FR GB IT LI LU NL SE**

1. DNA fragment containing a sequence of at most 318 pairs of nucleotides characterized in that it codes for a peptide containing all or part of the aminoacid sequence

```
Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser
```

it being understood that the sequence always contains the aminoacid sequence:

Asp Asn Pro Ala Ser Thr Thr Asn Lys Asp Lys Leu.

2. DNA fragment according to claim 1 characterized in that the sequence of 318 nucleotide pairs is characterized by the structure:

```
TCT AGA GAC GCT CTC CCA AAC ACT GAA
GCC AGT GGA CCA ACA CAC TCC AAG GAA ATT
CCG GCA CTC ACC GCA GTG GAA ACT GGG GCC
ACA AAT CCA CTA GTC CCT TCT GAT ACA GTG
CAA ACC AGA CAT GTT GTA CAA CAT AGG TCA
AGG TCA GAG TCT AGC ATA GAG TCT TTC TTC
GCG CGG GGT GCA TGC GTG ACC ATT ATG ACC
```

```
GTG GAT AAC CCA GCT TCC ACC ACG AAT AAG
GAT AAG CTA TTT GCA GTG TGG AAG ATC ACT
TAT AAA GAT ACT GTC CAG TTA CGG AGG AAA
TTG GAG TTC TTC ACC TAT TCT.
```

3. DNA fragment according to claim 1 or claim 2 characterized in that it contains the nucleotide sequence coding for the peptide sequence His 65-Phe 105:

```
                        His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe
100
```

4. DNA fragment according to claim 1 or claim 2 characterized in that it contains the nucleotide sequence:

```
                        His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe Ala Val Trp Lys
100
Ile
110
```

5. Fragment according to claim 1 or claim 2 characterized that it codes for the sequence:

Asp Asn Pro Ala Ser Thr Thr Asn Lys Asp Lys Leu.

6. Polypeptide containing all or part of the aminoacid sequence of the formula:

```
    Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
```

```
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser,
```

this polypeptide always containing the sequence:

Asp Asn Pro Ala Ser Thr Thr Asn Lys Asp Lys Leu.

7. Polypeptide according to claim 6 characterized in that it contains the aminoacid sequence of the formula:

```
                His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe
100
```

8. Polypeptide according to claim 6 characterized in that it contains the aminoacid sequence of the formula:

```
                His Val Val Gln His
Arg Ser Arg Ser Glu Ser Ser Ile Glu Ser
70
Phe Phe Ala Arg Gly Ala Cys Val Thr Ile
80
Met Thr Val Asp Asn Pro Ala Ser Thr Thr
90
Asn Lys Asp Lys Leu Phe Ala Val Trp Lys
100
Ile
110
```

9. Polypeptide according to claim 6 characterized by the aminoacid sequence of the formula:

Asp Asn Pro Ala Ser Thr Thr Asn Lys Asp Lys Leu.

24

10. Polypeptide according to claim 6 characterized in that it contains less than 20 aminoacid residues.

11. Polypeptide according to claim 6 characterized in that it contains less than 15 aminoacid residues.

12. Immunogenic conjugate resulting from the covalent coupling of a polypeptide according to any one of claims 6 to 11 with a carrrier molecule having preferably a molecular weight greater than 20,000.

13. Vector containing an insert constituted by a DNA sequence placed under the control of a promoter permitting the expression of this insert in a microorganism transformable by the vector characterized in that the sequence of this nucleotidic insert conforms to that of the fragment according to any one of claims 1 to 5.

14. Micro-organism transformed by the vector according to claim 13 and capable of producing a protein recognized by antibodies active both against particles "C" and "D" of a same poliovirus and against the VP-1 structural polypeptide of the capsid of this poliovirus.

15. Water soluble oligomer containing particularly from 2 to 10 monomeric units characterized in that the monomeric unit consists of a polypeptide such as defined in any one of claims 6 to 11.

16. Process for the preparation of a peptide such as defined in any one of claims 6 to 11 comprising the transformation of a micro-organism transformable by the vector according to claim 13 and in which the above said insert can be expressed under the control of the corresponding promoter, the recovery of the proteins synthesised and the isolation of the peptidic fraction containing the above said peptide whereby the latter can be detected possibly after a fractionation according to molecular weight by antibodies active both against "C" and "D" particles of a same poliovirus and against the VP-1 structural polypeptide of the capsid of this poliovirus.

17. Reagent for the diagnosis of the presence of antipoliomyolitic antibodies in a biological sample such as serum characterized in that it consists of an antigen adapted to react with these antibodies, this antigen consisting of a polypeptide according to any one of claims 6 to 11 or of an immunogenic conjugate according to claim 12 or furthermore of an immunogenic oligomer according to claim 15.

18. Probe for the detection of RNA of the polyomyelitis virus or of the corresponding cDNA containing a nucleotide sequence capable of hybridizing with said RNA or cDNA characterized in that said probe contains a DNA fragment according to any one of claims 1 to 5.

19. Use of a peptide according to any one of claims 1 to 5, of a conjugate according to claim 12 or of an oligomer according to claim 15 in the production of active parts of vaccines against polioviruses.

**Claims for the Contracting State: AT**

1. Process for the synthesis of a polypeptide containing all or part of the aminoacid sequence of the formula:

```
Ser Arg Asp Ala Leu Pro Asn Thr Glu
Ala Ser Gly Pro Thr His Ser Lys Glu Ile
Pro Ala Leu Thr Ala Val Glu Thr Gly Ala
Thr Asn Pro Leu Val Pro Ser Asp Thr Val
Gln Thr Arg His Val Val Gln His Arg Ser
Arg Ser Glu Ser Ser Ile Glu Ser Phe Phe
Ala Arg Gly Ala Cys Val Thr Ile Met Thr
Val Asp Asn Pro Ala Ser Thr Thr Asn Lys
Asp Lys Leu Phe Ala Val Trp Lys Ile Thr
Tyr Lys Asp Thr Val Gln Leu Arg Arg Lys
Leu Glu Phe Phe Thr Tyr Ser,
```

this polypeptide always containing the sequence:

Asp Asn Pro Ala Ser Thr Thr Asn Lys Asp Lys Leu

characterized by, starting preferably from the C-terminal aminoacid, successively condensing the successive aminoacyls groups two by two in the required order or condensing aminoacyl groups and fragments previously formed and containing already several aminoacyl residues in the appropriate order or further condensing several fragments previously prepared, it being understood that care is taken to protect beforehand all the reactive functions borne by these aminoacyl groups or fragments with the exception of the amine functions of the one and the carboxyl of the other or vice versa which must normally

25

take part in the formation of the peptide bonds particularly after activation of the carboxyl function according to methods known in the synthesis of peptides and so on in a stepwise manner until the N-terminal aminoacid.

2. Process according to claim 1 characterized in that the condensations are carried out until the aminoacid sequence of the formula:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe

100

is obtained.

3. Process according to claim 1 characterized in that the condensation is carried out until the aminoacid sequence of the formula:

His Val Val Gln His

Arg Ser Arg Ser Glu Ser Ser Ile Glu Ser

70

Phe Phe Ala Arg Gly Ala Cys Val Thr Ile

80

Met Thr Val Asp Asn Pro Ala Ser Thr Thr

90

Asn Lys Asp Lys Leu Phe Ala Val Trp Lys

100

Ile

110

4. Process according to claim 1 characterized in that the condensations are carried out until the aminoacid sequence of the formula:

Asp Asn Pro Ala Ser Thr Thr Asn Lys Asp Lys Leu

is obtained.

5. Process according to claim 1 characterized in that the condensations are carried out until a polypeptide containing less than 20 aminoacid residues is obtained.

6. Process according to claim 1 characterized in that the condensations are carried out until a polypeptide containing less than 15 aminoacid residues is obtained.

7. Process for the production of an immunogenic conjugate resulting from the covalent coupling of a polypeptide according to any one of claims 1 to 6 with a carrier molecule, preferably having a molecular weight greater than 20,000 and comprising one or more reactive carboxyl or amine functions characterized in that the peptide and the support molecule are reacted together in the presence of a coupling agent of the type used in the synthesis of proteins for example N-hydroxy-benzotriazole or in the presence of glutaraldehyde especially when the binding is between amine groups respectively carried by the peptide and the support molecule.

8. Process for the preparation of a water soluble oligomer containing particularly from 2 to 10 monomer units in which the monomeric unit consists of a polypeptide such as defined in any one of claims 1 to 6 characterized in that the monomers are reacted together in the presence of glutaraldehyde or any agent permitting the coupling of the separate reactive functions carried by each of the polypeptide monomers.

9. Process for the preparation of a peptide such as definied in any one of claims 1 to 6 containing an insert formed by a DNA sequence coding for one of the polypeptides such as obtained by the process according to any one of claims 1 to 6 under the control of a promoter and in which microorganism the said insert can be expressed under the control of the said promoter, the recovery of the proteins synthesized and the isolation of the peptide fraction containing the said peptide, whereby the latter can be detected, possibly after a fractionation according to molecular weights, by antibodies active both against "C" and "D" particles of a same poliovirus and against the VP-1 structural poliopeptide of the capside of this poliovirus.

10. Process for the diagnosis *in vitro* of the presence of antipoliomyelitic antibodies in a biological sample such as a serum characterized in that the sample is contacted with a polypeptide obtained by the process according to any one of claims 1 to 6 or 9 or with an immunogenic conjugate obtained by the process according to claim 7 or with the water soluble oligomer obtained by the process according to claim 8.

# Fig.1.

Fig.2.

```
     3210    3220      3230        3240         3250         3260           3270          3280          3290         3300
CTGTTAGAGTAGTCAATGATCACAACCCGACCCGACCAAGGTCACCCTCCAAAATCAGAGTGTATCTAAAACCCAAACACACTCAGAGTCTGGTGCCCGGCGTCCACC
                   BCL1                                                                                      BCER
                   SAU3A

     3310       3320       3330      3340        3350          3360           3370          3380          3390
GAGGGCAGTGGGCGTACGGCCCTGGAGTGGATTACAAGGATGGTACGGCTTACACCCCTCTCCACCAAGGATCTGACCACACATAT
      RSA1  HAEIII              RSA1                                                SAU3A        VP1
```

# Fig.3.

```
              2250     ·2260      2270       2280       2290       2300
        CTGCAGTCCTCATGTACTATGGTAGTGCCATGGATTAGCAACACCACGTATCGGCAAA
        ^              ^
        PST1           RSA1

    2310      2320      2330      2340      2350      2360      2370      2380      2390      2400
    CCATAGATGATAGTTTCACCGAAGGCGGATACATCAGCGTCTTCTACCAAACTAGAATAGTCGTCCCTCTTTCGACACCCAGAGAGATGGACATCCTTGG
                                                                          ^
                                                                          TAQ1

    2410      2420      2430      2440      2450      2460      2470↓     2480      2490      2500
    TTTTGTGTCAGCGTGTAATGACTTCAGCGTGCGCTTGTTGCGAGATACCACACATATAGAGCAAAAGCGCTAGCACAGGGTTAGGTCAGATGCTTGAA
                          ^                                               ^^
                          HHA1                                            HAE11          VP3 VP1
                                                                          HHA1

    2510      2520      2530      2540      2550      2560      2570      2580      2590      2600
    AGCATGATTGACAACACAGTCCGTGAAACGGTGGGGGCGGCAACATCTAGAGACGCTCTCCCAAACACTGAAGCCAGTGGACCAACACACTCCAAGGAAA
                                              ^                                His
                                              XBA1                             (65)

    2610      2620      2630      2640      2650      2660      2670      2680      2690      2700
    TTCCGGCACTCACCGCAGTGGAAACTGGGGCCACAAATCCACTAGTCCCTTCTGATACAGTGCAAACCAGACATGTTGTACAACATAGGTCAAGGTCAGA
    ^                             ^                                           ^              Phe
    HPA11                         HAE111                                      RSA1           (105)

    2710      2720      2730      2740      2750      2760      2770      2780      2790      2800
    GTCTAGCATAGAGTCTTTCTTCGCGCGGGGTGCATGCGTGACCATTATGACCGTGGATAACCCAGCTTCCACCACGAATAAGGATAAGCTATTTGCAGTG
                        ^^^                                     ^                        ^
                        BCER                                    ALU1                     ALU1
                        HHA1
                        BCER

    2810      2820      2830      2840      2850      2860      2870      2880      2890      2900
    TGGAAGATCACTTATAAAGATACTGTCCAGTTACGGAGGAAATTGGAGTTCTTCACCTATTCTAGATTTGATATGGAACTTACCTTTGTGGTTACTGCAA
    ^                                                      ^
    SAU3A                                                  XBA1
```

EP 0 110 791 B1

# Fig.4.

```
     2910      2920      2930      2940      2950      2960       2970      2980      2990      3000
ATTTCACTGAGACTAACAATGGGCATGCCTTAAATCAAGTGTACCAAATTATGTACGTACCACCAGGCGCTCCAGTGCCCGAGAAATGGGACGACTACAC
                                        ^              ^     ^            ^^            ^
                                       RSA1           RSA1              HAE11        AVA1
                                                        RSA1            HHA1
                                                                         HHA1

     3010      3020      3030      3040      3050      3060       3070      3080      3090      3100
ATGGCAAACCTCATCAAATCCATCAATCTTTTACACCTACGGAACAGCTCCAGCCCGGATCTCGGTACGGTATGTTGGTATTTCGAACGCCTATTCACAC
                                        ^              ^   ^      ^^                    ^^
                                       ALU1          HPA1   KPN1                      ASU11
                                                      SAU3A RSA1                       TAQ1

     3110      3120      3130      3140      3150      3160       3170      3180      3190      3200
TTTTACGACGGTTTTTCCAAAGTACCACTGAAGGACCAGTCGGCAGCACTAGGTGACTCCCTTTATGGTGCAGCATCTCTAAATGACTTCGGTATTTTGG
            ^
           RSA1

     3210      3220      3230      3240      3250      3260       3270      3280      3290      3300
CTGTTAGAGTAGTCAATGATCACAACCCGACCAAGGTCACCTCCAAAATCAGAGTGTATCTAAAACCCAAACACATCAGAGTCTGGTGCCCGCGTCCACC
                 ^^                                                                            ^
                BCL1                                                                          BCER
                SAU3A

     3310      3320      3330      3340      3350      3360       3370      3380      | 3390     3400
GAGGGCAGTGGCGTACTACGGCCCTGGAGTGGATTACAAGGATGGTACGCTTACACCCCTCTCCACCAAGGATCTGACCACATAGGATTCGGACACCAA
           ^          ^                            ^                          ^           └─
          RSA1      HAE111                       RSA1                       SAU3A        VP1

     3410      3420
AACAAAGCGGTGTACACTGCAGG
          ^     ^
         RSA1  PST1
```

EP 0 110 791 B1

EP 0 110 791 B1

Fig.5a.

Fig.5b.

Fig.5c.

Fig.5d.

Fig.5e.

Fig.5f.

Fig 5g

Fig.5h.

5

Fig.6a.

Fig.6b.

Fig.6c.

Fig.6d.

Fig.6e.

Fig.6f.

Fig. 7.

EP 0 110 791 B1

# Fig.8.

```
LEU GLN SER SER CYS THR MET VAL VAL PRO TRP ILE SER ASN THR THR TYR ARG GLN THR
GTG CAG TCC TCA TGT ACT ATG GTA GTG CCA TGG ATT AGC AAC ACC ACG TAT CGG CAA ACC
23C3 PstI
ILE ASP ASP SER PHE THR GLU GLY GLY TYR ILE SER VAL PHE TYR GLN THR ARG ILE VAL
ATA GAT GAT AGT TTC ACC GAA GGC GGA TAC ATC AGC GTC TTC TAC CAA ACT AGA ATA GTC
2363
VAL PRO LEU SER THR PRO ARG GLU MET ASP ILE LEU GLY PHE VAL SER ALA CYS ASN ASP
GTC CCT CTT TCG ACA CCC AGA GAG ATG GAC ATC CTT GGT TTT GTG TCA GCG TGT AAT GAC
2423                                  ← VP3 →                          VP3 ← VP1
PHE SER VAL ARG LEU LEU ARG ASP THR THR HIS ILE GLU GLN LYS ALA LEU ALA GLN GLY
TTC AGC GTG CGC TTG TTG CGA GAT ACC ACA CAT ATA GAG CAA AAA GCG CTA GCA CAG GGG
2483
LEU GLY GLN MET LEU GLU SER MET ILE ASP ASN THR VAL ARG GLU THR VAL GLY ALA ALA
TTA GGT CAG ATG CTT GAA AGC ATG ATT GAC AAC ACA GTC CGT GAA ACG GTG GGG GCG GCA
2543                                  ·
THR SER ARG ASP ALA LEU PRO ASN THR GLU ALA SER GLY PRO THR HIS SER LYS GLU ILE
ACA TCT AGA GAC GCT CTC CCA AAC ACT GAA GCC AGT GGA CCA ACA CAC TCC AAG GAA ATT
2603  XbaI
PRO ALA LEU THR ALA VAL GLU THR GLY ALA THR ASN PRO LEU VAL PRO SER ASP THR VAL
CCG GCA CTC ACC GCA GTG GAA ACT GGG GCC ACA AAT CCA CTA GTC CCT TCT GAT ACA GTG
2663
GLN THR ARG HIS VAL VAL GLN HIS ARG SER ARG SER GLU SER SER ILE GLU SER PHE PHE
CAA ACC AGA CAT GTT GTA CAA CAT AGG TCA AGG TCA GAG TCT AGC ATA GAG TCT TTC TTC
2723
ALA ARG GLY ALA CYS VAL THR ILE MET THR VAL ASP ASN PRO ALA SER THR THR ASN LYS
GCG CGG GGT GCA TGC GTG ACC ATT ATG ACC GTG GAT AAC CCA GCT TCC ACC ACG AAT AAG
2783
ASP LYS LEU PHE ALA VAL TRP LYS ILE THR TYR LYS ASP THR VAL GLN LEU ARG ARG LYS
GAT AAG CTA TTT GCA GTG TGG AAG ATC ACT TAT AAA GAT ACT GTC CAG TTA CGG AGG AAA
2843  ·
LEU GLU PHE PHE THR TYR SER ARG PHE ASP MET GLU LEU THR PHE VAL VAL THR ALA ASN
TTG GAG ITC TTC ACC TAT TCT AGA TTT GAT ATG GAA CTT ACC TTT GTG GTT ACT GCA AAT
2903  .                 XbaI                              ..
PHE THR GLU THR ASN ASN GLY HIS ALA LEU ASN GLN VAL TYR GLN ILE MET TYR VAL PRO
TTC ACT GAG ACT AAC AAT GGG CAT GCC TTA AAT CAA GTG TAC CAA ATT ATG TAC GTA CCA
2963
PRO GLY ALA PRO VAL PRO GLU LYS TRP ASP ASP TYR THR TRP GLN THR SER SER ASN PRO
CCA GGC GCT CCA GTG CCC GAA AAA TGG GAC GAC TAC ACA TGG CAA ACC TCA TCA AAT CCA
3023
SER ILE PHE TYR THR TYR GLY THR ALA PRO ALA ARG ILE SER VAL PRO TYR VAL GLY ILE
TCA ATC TTT TAC ACC TAC GGA ACA GCT CCA GCC CGG ATC TCG GTA CCG TAT GTT GGT ATT
3083                                             KpnI
SER ASN ALA TYR SER HIS PHE TYR ASP GLY PHE SER LYS VAL PRO LEU LYS ASP GLN SER
TCG AAC GCC TAT TCA CAC TTT TAC GAC GGT TTT TCC AAA GTA CCA CTG AAG GAC CAG TCG
3143
ALA ALA LEU GLY ASP SER LEU TYR GLY ALA ALA SER LEU ASN ASP PHE GLY ILE LEU ALA
GCA GCA CTA GGT GAC TCC CTT TAT GGT GCA GCA TCT CTA AAT GAC TTC GGT ATT TTG GCT
3203
VAL ARG VAL VAL ASN ASP HIS ASN PRO THR LYS VAL THR SER LYS ILE ARG VAL TYR LEU
GTT AGA GTA GTC AAT GAT CAC AAC CCG ACC AAG GTC ACC TCC AAA ATC AGA GTG TAT CTA
3263
LYS PRO LYS HIS ILE ARG VAL TRP CYS PRO ARG PRO PRO ARG ALA VAL ALA TYR TYR GLY
AAA CCC AAA CAC ATC AGA GTC TGG TGC CCG CGT CCA CCG AGG GCA GTG GCG TAC TAC GGC
3323
PRO GLY VAL ASP TYR LYS ASP GLY THR LEU THR PRO LEU SER THR LYS ASP LEU THR THR
CCT GGA GTG GAT TAC AAG GAT GGT ACG CTT ACA CCC CTC TCC ACC AAG GAT CTG ACC ACA
VP1 3383      35     ← NCNP 3b →
TYR GLY PHE GLY HIS GLN ASN LYS ALA VAL TYR THR ALA GLY TYR LYS ILE CYS ASN TYR
TAT GGA TTC GGA CAC CAA AAC AAA GCG GTG TAC ACT GCA GGT TAC AAA ATT TGC AAC TAC
3443              PstI
HIS LEU ALA THR GLN ASP ASP LEU GLN ASN ALA VAL ASN VAL MET TRP SER ARG ASP LEU
CAC TTG GCC ACT CAG GAT GAT TTG CAA AAC GCA GTG AAC GTC ATG TGG AGT AGA GAC CTC
3503
LEU VAL THR GLU SER ARG ALA GLN GLY THR ASP SER ILE ALA ARG CYS ASN CYS ASN ALA
TTA GTC ACA GAA TCA AGA GCC CAG GGC ACC GAT TCA ATC GCA AGG TGC AAT TGC AAC GCA
3563
GLY VAL TYR TYR CYS GLU SER ARG ARG LYS TYR TYR PRO VAL SER PHE VAL GLY PRO THR
GGG GTG TAC TAC TGC GAG TCT AGA AGG AAA TAC TAC CCA GTA TCC TTC GTT GGC CCA ACG
```

8

# Fig. 9.

# Fig. 10.